# EUROPÄISCHE PATENTSCHRIFT

(11) **EP 0 433 780 B1**
(45) Veröffentlichungstag und Bekanntmachung des Hinweises auf die Patenterteilung: **03.04.1996**
(21) Anmeldenummer: 90123378.3
(22) Anmeldetag: 06.12.1990
(51) Int. Cl.: C07D 249/08, C07D 233/60, C07D 401/12, C07D 405/08, C07D 401/08, C07D 405/14, C07D 409/08, C07D 403/08, C07D 409/14, C07D 401/14, C07D 405/06, C07D 409/06, C07D 303/22, C07D 303/08, C07D 405/12, C07D 303/34, C07D 407/06, C07D 303/04, C07D 407/04, C07D 409/04, C07D 405/04, A01N 43/50, A01N 43/653

(54) **Substituierte Azolylmethylcycloalkanole und diese enthaltende Fungizide**
Substituted azolylmethylcycloalkanols and fungicides containing them
Azolylméthylcycloalkanols substitués et fongicides les contenant

(30) Priorität: 16.12.1989 DE 3941593; 27.06.1990 DE 4020432; 18.07.1990 DE 4022784; 14.09.1990 DE 4029197
(43) Veröffentlichungstag der Anmeldung: 26.06.1991
(73) Patentinhaber: BASF Aktiengesellschaft, D-67063 Ludwigshafen (DE)
(72) Erfinder: Seele, Rainer, Dr., W-6701 Fussgoenheim (DE); Zipperer, Bernhard, Dr., W-6716 Dirmstein (DE); Keil, Michael, Dr., W-6713 Freinsheim (DE); Goetz, Norbert, Dr., W-6520 Worms 1 (DE); Ammermann, Eberhard, Dr., W-6700 Ludwigshafen (DE); Lorenz, Gisela, Dr., W-6730 Neustadt (DE); Kober, Reiner, Dr., W-6701 Fussgoenheim (DE); Kuekenhoehner, Thomas, Dr., W-6710 Frankenthal (DE); Harreus, Albrecht, Dr., W-6700 Ludwigshafen (DE); Rademacher, Wilhelm, Dr., W-6703 Limburgerhof (DE)

(56) Entgegenhaltungen:
- EP-A- 0 052 425
- EP-A- 0 259 814
- EP-A- 0 267 778
- EP-A- 0 272 895
- EP-A- 0 324 646
- EP-A- 0 374 509
- EP-A- 0 378 953
- DE-A- 3 617 071
- DE-A- 3 902 031
- FR-A- 2 472 559
- US-A- 4 414 210
- JOURNAL OF ORGANIC CHEMISTRY, Band 46, Nr. 1, 1981, US; F.E. ZIEGLER et al., Seiten 122-128
- JOURNAL OF THE AMERICAN CHEMICAL SOCIETY, Band 111, 1989, US; E. VEDEJS et al., Seiten 6861-6862
- BULLETIN DE LA SOCIETE CHIMIQUE DE FRANCE, Nr. 5-6, Mai/Juni 1974, Paris (FR); A. SEVIN et al., Seiten 963-969
- GAZETTA CHIMICA ITALIANA, Band 113, Nr. 11-12, 1983, IT; G. DI MAIO et al., Seiten 823-827
- CHEMICAL & PHARMACEUTICAL BULLETIN, Band 20, Nr. 12, Dezember 1972, JP; S. OIDA et al., Seiten 2634-2641
- PHOSPHORUS & SULFUR, Band 19, 1984, US; N. SATYAMURTHY et al., Seiten 113-129
- JOURNAL OF THE INDIAN CHEMICAL SOCIETY, Band 38, Nr. 8, August 1961; T.M. JACOB et al., Seiten 674-678
- JOURNAL OF THE AMERICAN CHEMICAL SOCIETY, Band 95, Nr. 2, 24 Januar 1973, US; B.L. SHAPIRO et al., Seiten 520-526
- TETRAHEDRON LETTERS, Band 41, Nr. 5, 1985, GB; A. SCHNEIDER et al., Seiten 949-953
- CHEMISCHE BERICHTE, Band 96, Nr. 7, 1963, Weinheim (DE); V. FRANZEN et al., Seiten 1881-1890
- JOURNAL OF THE AMERICAN CHEMICAL SOCIETY, Band 87, Nr. 6, 20 März 1965, US; E.J. COREY et al., Seiten 1353-1364
- JOURNAL OF ORGANIC CHEMISTRY, Band 51, Nr. 20, 1986, US; S. SHIMIZU et al., Seiten 3897-3900
- JOURNAL OF THE CHEMICAL SOCIETY - PERKIN TRANSACTIONS I, 1990; A.L. BECK et al., Seiten 689-693

## Beschreibung

Die vorliegende Erfindung betrifft neue Azolylmethylcycloalkanole der allgemeinen Formel I 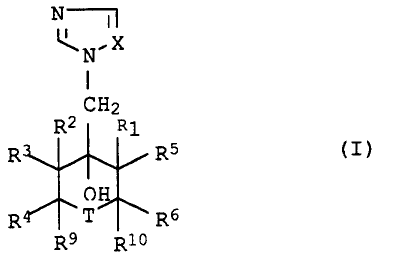
in der
- X: CH oder N bedeutet;
- T: (CH₂)ₙ, CR¹¹R¹², O oder S bedeutet,
wobei
- n: eine ganze Zahl von 1 bis 5 bedeutet; und
- R¹¹, R¹²: Wasserstoff oder C₁-C₈-Alkyl, Phenyl, Biphenyl, Naphthyl, Heteroaryl, Benzyl, Dioxolanyl oder C₃-C₈-Cycloalkyl bedeutet, wobei jeder dieser Reste ein- bis dreifach durch Halogen, Nitro, Phenoxy, Amino, C₁-C₄-Alkyl, c₁-C₄-Alkoxy oder c₁-c₄-Halogenalkyl substituiert sein kann;
- R¹ und R⁵: gleich oder verschieden sind und für Wasserstoff oder C₁-C₄-Alkyl stehen oder
- R¹ und R⁵: gemeinsam mit dem C-Atom, dessen Substituenten sie sind, für C=CH-R⁷ oder für CH-Z-R⁷ stehen,
wobei
- Z: CH₂, O, S, SO, SO₂ oder N-R⁸ bedeutet,
- R⁷: C₁-C₈-Alkyl, Phenyl, Biphenyl, Naphthyl, Heteroaryl, Benzyl oder C₃-C₈-Cycloalkyl bedeutet, wobei jeder dieser Reste ein- bis dreifach durch Halogen, Nitro, Phenoxy, Amino, C₁ -C₄-Alkyl, C₁-C₄-Alkoxy oder C₁-C₄-Halogenalkyl substituiert sein kann; oder Tetrahydropyranyl bedeutet; wobei
- R⁷: für den Fall, daß R¹ und R⁵ gemeinsam mit dem C-Atom, dessen Substituenten sie sind, eine CH-Z-R⁷-Gruppe bedeutet, zusätzlich für Wasserstoff stehen kann; und
- R⁸: für Wasserstoff oder C₁-C₄-Alkyl steht;
- R² und R³: jeweils für Wasserstoff oder C₁-C₄-Alkyl stehen; oder
- R² und R³: gemeinsam mit dem C-Atom, dessen Substituenten sie sind, eine C=CH-R⁷ Gruppe, für den Fall, daß R¹ und R⁵ gemeinsam mit dem C-Atom, dessen Substituenten sie sind, eine C=CH-R⁷ Gruppe bedeuten;
- R⁴: Wasserstoff oder C₁-C₄-Alkyl
und
- R⁶,R⁹ u.R¹⁰: Wasserstoff oder
C₁-C₈-Alkyl, Phenyl, Biphenyl, Naphthyl, Heteroaryl, Benzyl, Dioxolanyl oder C₃-C₈-Cycloalkyl bedeutet, wobei jeder dieser Reste ein- bis dreifach durch Halogen, Nitro, Phenoxy, Amino, C₁-C₄-Alkyl, C₁-C₄-Alkoxy oder C₁-C₄-Halogenalkyl substituiert sein kann;
ausgenommen Verbindungen,
a) in denen die Reste R¹ bis R¹² gleichzeitig Wasserstoff bedeuten,
b) in denen R¹ und R⁵ gemeinsam mit dem C-Atom, an das sie gebunden sind, für CH-CH₂-(4-Chlorphenyl) oder CH-O-(4-Chlorphenyl) stehen, wenn R² bis R⁴ und R⁶ bis R¹² Wasserstoff bedeuten,
c) in denen R¹ und R⁵ gemeinsam mit dem C-Atom,an das sie gebunden sind, für C=CH-(4-Fluorphenyl) oder für C=CH-(4-Chlorphenyl) stehen, wenn
   - T: CH₂ oder (CH₂)₂ bedeutet,
   - R² und R³: für Wasserstoff, Cyclohexyl, Phenyl oder C₁-C₄-Alkyl stehen und
   - R⁴,R⁶,R⁹ und R¹⁰: Wasserstoff bedeuten;
sowie deren pflanzenverträgliche Säureadditionssalze und Metallkomplexe.

Außerdem betrifft die Erfindung ein Verfahren zur Herstellung dieser Verbindungen, fungizide und wachstumsregulatorische Mittel, welche die erfindungsgemäßen Azolylmethylcycloalkanole als wirksame Substanzen enthalten, sowie Verfahren zur Bekämpfung von Pilzen und Verfahren zur Regulierung des Pflanzenwachstums mit Hilfe dieser Verbindungen.

Aus der EP-A-324 646 sind azolsubstituierte Cycloalkanolderivate und ihre Verwendung als Fungizide bekannt, die als Rest -Y-R¹ eine Gruppe 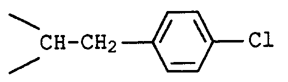
tragen. Weitere, teilweise fungizide, Verbindungen ähnlicher Strukturen sind der US-A 4 414 210, der JP-A 01/186 870, der EP-A-378 953 sowie J. Org. Chem. 51, 3897 (1986) zu entnehmen. Die fungiziden Wirkungen sind jedoch nicht in allen Fällen befriedigend.

Der Erfindung lag daher die Aufgabe zugrunde, neue Azolverbindungen mit besseren fungiziden und wachstumsregulatorischen Wirkungen bereitzustellen.

Demgemäß wurden die eingangs definierten Azolylmethylcycloalkanole der allgemeinen Formel I sowie deren pflanzenverträgliche Säureadditionssalze und Metallkomplexe gefunden.

Weiterhin wurden Verfahren zur Herstellung dieser Verbindungen, fungizide und wachstumsregulatorische Mittel, welche die erfindungsgemäßen Azolylmethylcycloalkanole als wirksame Substanzen enthalten, sowie Verfahren zur Bekämpfung von Pilzen und Verfahren zur Regulierung des Pflanzenwachstums mit Hilfe dieser Verbindungen gefunden.

Die Azolylmethylcycloalkanole der allgemeinen Formel I erhält man i.a. in Form von Racematen bzw. als Diastereomerengemische. Diese Isomeren lassen sich in üblicher Weise, beispielsweise aufgrund ihrer Löslichkeit oder der ihrer Salze oder auch durch Säulenchromatographie, trennen und in reiner Form isolieren. Aus einem solchen isolierten Diastereomeren kann man mit bekannten Methoden einheitliche Enantiomere erhalten.

Als fungizide Wirkstoffe können sowohl die einzelnen Diastereomere bzw. Enantiomere als auch deren bei der Synthese i.a. anfallenden Gemische verwendet werden.

Von den im folgenden angeführten, im Hinblick auf ihre fungizide Wirkung bevorzugten Verbindungen I sind diejenigen Verbindungen ausgenommen,
a) in denen die Reste R¹ bis R¹² gleichzeitig Wasserstoff bedeuten,
b) in denen R¹ und R⁵ gemeinsam mit dem C-Atom, an das sie gebunden sind, für CH-CH₂-(4-Chlorphenyl) oder CH-O-(4-Chlorphenyl) stehen, wenn R² bis R⁴ und R⁶ bis R¹² Wasserstoff bedeuten,
c) in denen R¹ und R⁵ gemeinsam mit dem C-Atom, an das sie gebunden sind, für C=CH-(4-Fluorphenyl) oder für C=CH-(4-Chlorphenyl) stehen, wenn
   - T: CH₂ oder (CH₂)₂ bedeutet,
   - R² und R³: für Wasserstoff, Cyclohexyl, Phenyl oder C₁- C₄-Alkyl stehen und
   - R⁴,R⁶,R⁹ und R¹⁰: Wasserstoff bedeuten;
sowie deren pflanzenverträgliche Säureadditionssalze und Metallkomplexe.

Diejenigen Verbindungen I sind besonders bevorzugt, in denen R¹ und R⁵ gemeinsam mit dem C-Atom, dessen Substituenten sie sind, für eine >C=CH-Gruppe stehen.

Für den Fall, daß R¹ und R⁵ gemeinsam mit dem C-Atom, dessen Substituenten sie sind, eine >C=CH-Gruppe bedeutet, sind zusätzlich die Verbindungen bevorzugt, in denen R⁷ 4-Tetrahydropyranyl darstellt.

Stehen R¹ und R⁵ gemeinsam mit dem C-Atom, dessen Substituenten sie sind, für eine >CH-Z-Gruppe, sind die Verbindungen I bevorzugt, in denen Z für 0 oder S steht.

Für den Fall, daß R¹ und R⁵ gemeinsam mit dem C-Atom, dessen Substituenten sie sind, eine >CH-Z-Gruppe bedeuten, sind zusätzlich noch die erfindungsgemäßen Verbindungen der Formel I bevorzugt, in denen R⁷ ein Wasserstoffatom darstellt.

Weiterhin bevorzugt sind Verbindungen I, in denen R⁷ einen ggf. ein- bis dreifach durch Fluor oder Chlor substituierten Phenylrest bedeuten.

Bevorzugt ist ferner
eine Verbindung I, in der X für CH steht, R¹ und R⁵ gemeinsam mit dem C-Atom, an das sie gebunden sind, für CH-S-(4-Chlorphenyl) stehen, T für CH₂ steht und R² bis R⁴ und R⁶, R⁹ und R¹⁰ Wasserstoff bedeuten,
eine Verbindung I, in der X für CH steht, R¹ und R⁵ sowie R² und R³ jeweils gemeinsam mit dem C-Atom, an das sie gebunden sind, für C=CH-(4-Chlorphenyl) stehen, T für CH₂ steht und R⁴, R⁶, R⁹ und R¹⁰ Wasserstoff bedeuten und
eine Verbindung I, in der X für N steht, R¹ und R⁵ sowie R² und R³ jeweils gemeinsam mit dem C-Atom, an das sie gebunden sind, für C=CH-(4-Chlorphenyl) stehen, T für CH₂ steht und R⁴, R⁶, R⁹ und R¹⁰ Wasserstoff bedeuten.

Die Reste R² und R³ können unabhängig voneinander gleich oder verschieden sein, sie stehen vorzugsweise jeweils für ein Wasserstoffatom oder einen n-Alkylrest mit 1 bis 4 C-Atomen, insbesondere Methyl. Für den Fall, daß R¹ und R⁵ gemeinsam mit dem C-Atom, dessen Substituenten sie sind, eine >CH-Z-Gruppe bedeuten, sind die erfindungsgemäßen Verbindungen I bevorzugt, bei denen die Reste R² und R³ gleich sind. Stehen R¹ und R⁵ gemeinsam mit dem C-Atom, dessen Substituenten sie sind, für eine >C=CH-Gruppe, sind insbesondere die Azolylmethylcycloalkanole I zu nennen, bei denen die Reste R² und R³ gemeinsam mit dem C-Atom, dessen Substituenten sie sind, >C=CH-R⁷ bedeuten. Es steht dann vorzugsweise für tert.-Butyl, Phenyl, 2-Chlorphenyl, 4-Chlorphenyl, 4-Fluorphenyl, 2-Bromphenyl, 4-Bromphenyl, 2,4-Dichlorphenyl, 2-Methylphenyl, 4-Methylphenyl, 2-Methoxyphenyl, 4-Methoxyphenyl sowie 2-Trifluormethylphenyl und 4-Trifluormethylphenyl, ferner 2-Furyl, Cyclopentyl und Cyclohexyl.

Bevorzugt werden Azolylmethylcycloalkanole der allgemeinen Formel 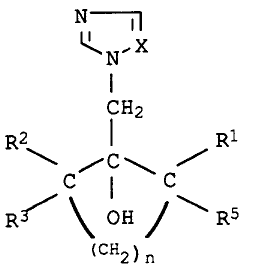
in welcher
R¹ und R⁵ gemeinsam mit dem C-Atom, dessen Substituenten sie sind, für C=CH-R⁷ oder CH-Z-R⁷ stehen,
wobei Z O, S, SO, SO₂ oder N-R⁸ bedeutet;
R⁷
C₁-C₈-Alkyl, Phenyl, Biphenyl, Naphthyl, Heteroaryl, Benzyl oder C₃-C₈-Cycloalkyl bedeutet, wobei jeder dieser Reste einfach bis dreifach durch Halogen, Nitro, Phenoxy, Amino, C₁-C₄-Alkyl, C₁-C₄-Alkoxy oder C₁- bis C₄-Halogenalkyl substituiert sein kann, oder Tetrahydropyranyl bedeutet,
oder, für den Fall, daß R¹ und R⁵ gemeinsam mit dem C-Atom, dessen Substituenten sie sind, eine >CH-Z-R⁷-Gruppe bedeuten, zusätzlich für ein Wasserstoffatom stehen kann;
R⁸
für Wasserstoff oder C₁-C₄-Alkyl steht;
R² und R³
jeweils für ein Wasserstoffatom oder eine C₁-C₄-Alkylgruppe stehen; oder, für den Fall, daß R¹ und R⁵ gemeinsam mit dem C-Atom, dessen Substituenten sie sind, eine >C=CH-R⁷-Gruppe bedeuten, gemeinsam mit dem C-Atom, dessen Substituenten sie sind, >C=CH-R⁷ bedeuten;
n
den Wert einer ganzen Zahl von 2 bis 5 annimmt;
X
den Rest CH oder N bedeutet;
sowie deren pflanzenverträgliche Säureadditionssalze und Metallkomplexe.

Bevorzugt werden ferner Azolylethanolderivate der allgemeinen Formel I 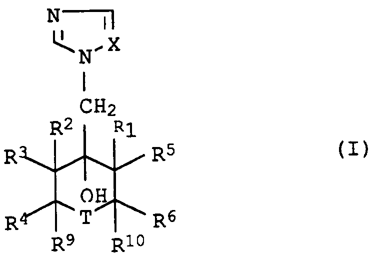
in welcher
R¹ und R⁵ gemeinsam mit dem C-Atom, dessen Substituenten sie sind, für C=CH-R⁷ oder CH-CH₂-R⁷ stehen;
R⁷ C₁-C₈-Alkyl, Phenyl, Biphenyl, Naphthyl, Heteroaryl, Benzyl oder C₃-C₈-Cycloalkyl bedeutet, wobei jeder dieser Reste einfach bis dreifach durch Halogen, Nitro, Phenoxy, Amino, C₁-C₄-Alkyl, C₁-C₄-Halogenalkyl substituiert sein kann;
R², R³, R⁴ gleich oder verschieden sind und für Wasserstoff oder C₁-C₄-Alkyl stehen; oder,
für den Fall, daß R¹ und R⁵ gemeinsam mit dem C-Atom, dessen Substituenten sie sind, eine >C=CH-R⁷-Gruppe bedeuten, zusätzlich R² und R³ gemeinsam mit dem C-Atom, dessen Substituenten sie sind, auch >C=CH-R⁷ bedeuten,
T den Rest CH₂, O oder S bedeutet,
X den Rest CH oder N bedeutet,
sowie deren pflanzenverträgliche Säureadditionssalze und Metallkomplexe.

Bevorzugt werden ferner Azolylmethylcycloalkanole der allgemeinen Formel I 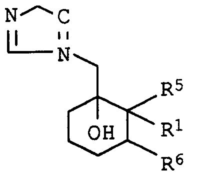
in welcher
R¹ und R⁵
gleich oder verschieden sind und für Wasserstoff oder C₁- bis C₄-Alkyl stehen;
R⁶
C₁-C₈-Alkyl, Phenyl, Biphenyl, Naphthyl, Heteroaryl, Benzyl, Dioxolanyl oder C₃-C₈-Cycloalkyl bedeutet, wobei jeder dieser Reste einfach bis dreifach durch Halogen, Nitro, Phenoxy, Amino, C₁-C₄-Alkyl, C₁-C₄-Alkoxy oder C₁-C₄-Halogenalkyl substituiert sein kann;
X
den Rest CH oder N bedeutet,
sowie deren pflanzenverträglichen Säureadditionssalze und Metallkomplexe.

Bevorzugt werden ferner Azolylmethylcyclohexanole der allgemeinen Formel I 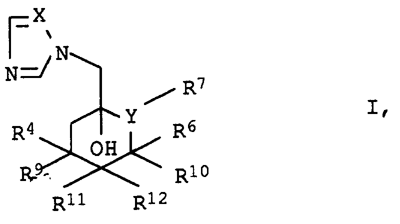
in welcher
Y für C=CH- oder CH-CH₂- steht;
R⁴
Wasserstoff oder C₁-C₄-Alkyl bedeutet,
R⁹ bis R¹²
Wasserstoff, C₁-C₈-Alkyl, C₃-C₆-Cycloalkyl oder Phenyl bedeutet, wobei jeder dieser Reste einfach bis dreifach durch Halogen, Nitro, Phenoxy, Amino, C₁-C₄-Alkyl, C₁-C₄-Alkoxy oder C₁-C₄-Halogenalkyl substituiert sein kann,
R⁷
C₁-C₈-Alkyl, Phenyl, Diphenyl, Naphthyl, Heteroaryl, Benzyl oder C₃-C₆-Cycloalkyl bedeutet, wobei jeder dieser Reste einfach bis dreifach durch Halogen, Nitro, Phenoxy, Amino, C₁-C₄-Alkoxy oder C₁-C₄-Halogenalkyl substituiert sein kann;
X
den Rest CH oder N bedeutet,
sowie deren pflanzenverträgliche Säureadditionssalze und Metallkomplexe.

Bevorzugt sind ferner die Verbindungen der allgemeinen Formel I, in der T CH₂ bedeutet, mit der Maßgabe, daß R⁴ und R⁹ nicht beide gleichzeitig Wasserstoff bedeuten.

Für den Fall, daß R¹ und R⁵ gemeinsam mit dem C-Atom, dessen Substituenten sie sind, eine >C=CH-Gruppe bedeuten, ist der Rest R⁷ an die >CH-Gruppe gebunden. Stehen R¹ und R⁵ gemeinsam mit dem C-Atom, dessen Substituenten sie sind, für eine >CH-Z-Gruppe, so liegt eine Bindung zwischen R⁷ und Z vor.

Die einzelnen Reste haben beispielsweise folgende Bedeutungen:
C₁- bis C₈-Alkyl, vorzugsweise C₁- bis C₄-Alkyl, insbesondere Methyl, Ethyl, n-Propyl, Isopropyl, n-Butyl, sec.-Butyl und tert.-Butyl; von den C₁- bis C₈-Alkylresten, die mehr als 4 C-Atome aufweisen, sind als bevorzugt n-Pentyl und Neopentyl zu nennen;
Phenyl und halogensubstituiertes Phenyl wie 2-Chlorphenyl, 3-Chlorphenyl, 4-Chlorphenyl, 2-Fluorphenyl, 3-Fluorphenyl, 4-Fluorphenyl sowie 2-Bromphenyl, 3-Bromphenyl und 4-Bromphenyl, 2,3-Dichlorphenyl, 2,4-Dichlorphenyl, 2,5-Dichlorphenyl und 2,6-Dichlorphenyl sowie 2-Chlor-4-fluorphenyl und 2-Chlor-6-fluorphenyl;
einfach durch Nitro-, Phenoxy-, Amino- und C₁- bis C₄-Alkylgruppen substituiertes Phenyl, wie 3-Nitrophenyl, 4-Nitrophenyl, 3-Phenoxyphenyl, 4-Phenoxyphenyl, 3-Aminophenyl und 4-Aminophenyl sowie 4-Ethylphenyl, 4-Isopropylphenyl und 4-tert.-Butylphenyl;
durch zwei oder drei der bereits erwähnten, aber verschiedenartigen Reste substituiertes Phenyl, wie 2-Chlor-6-methylphenyl;
einfach oder zweifach durch C₁- bis C₄-Alkoxygruppen substituiertes Phenyl, wie 2-Methoxyphenyl, 3-Methoxyphenyl, 4-Methoxyphenyl, 4-tert.-Butyloxyphenyl und 2,4-Dimethoxyphenyl sowie 3,4-Dimethoxyphenyl;
dreifach halogensubstituiertes Methylphenyl, wie 2-Trifluormethyl-, 3-Trifluormethyl- und 4-Trifluormethylphenyl;
p-Biphenyl;
1-Naphthyl und 2-Naphthyl;
Heteroaryl mit 5 oder 6 Ringatomen, wobei Ringe mit 6 Atomen mit bis zu drei Stickstoffatomen, wie 2-Pyridyl, 3-Pyridyl und 4-Pyridyl insbesondere zu nennen sind, sowie Ringe mit 5 Atomen mit bevorzugt einem oder zwei der Heteroatome O, S und N, insbesondere 2-Furyl, 2-Thienyl, 3-Thienyl, 4-Oxazolyl, 4-Thiazolyl, 4-Isoxazolyl, 5-Isoxazolyl und 5-Imidazolyl;
Benzyl; Halogenbenzyl;
C₃- bis C₈-Cycloalkyl, vorzugsweise Cyclopentyl und Cyclohexyl.

Als Säureadditionssalze eignen sich die pflanzenverträglichen Salze von solchen Säuren, welche die fungizide Wirkung von 1 nicht beeinträchtigen, also z.B. die Hydrochloride, Bromide, Sulfate, Nitrate, Phosphate, Oxalate und Dodecylbenzolsulfonate. Da die Wirksamkeit der Salze jedoch auf das Kation zurückgeht, kommt es auf das Anion i.a. nicht an. Die erfindungsgemäßen Wirkstoffsalze werden zweckmäßigerweise durch Umsetzung der Azolylmethylcycloalkanole I mit geeigneten Säuren hergestellt.

Metallkomplexe der erfindungsgemäßen Verbindungen I oder ihrer Salze werden bevorzugt mit Metallen der 11. Hauptgruppe wie Magnesium oder Calcium, der III. und IV. Hauptgruppe wie Aluminium, Zinn oder Blei oder mit Metallen der I. bis VIII.

Nebengruppe gebildet, wobei die Nebengruppenelemente der 4. Periode besonders bevorzugt sind, insbesondere Kupfer, Zink, Mangan, Eisen, Kobalt und Nickel. Hierzu werden die Azolylmethylcycloalkanole I mit den entsprechenden Metallsalzen umgesetzt.

Die Herstellung der erfindungsgemäßen Azolylmethylcycloalkanole I erfolgt i.a. durch Umsetzung einer Verbindung der allgemeinen Formel II mit einer Verbindung der allgemeinen Formel III. 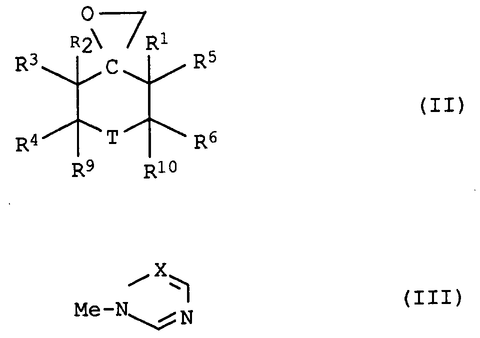
Von den Verbindungen III sind diejenigen bevorzugt, in denen Me für ein Wasserstoffatom oder ein Alkalimetallatom, insbesondere Natrium oder Kalium steht.

Falls Me ein Wasserstoffatom bedeutet, wird zweckmäßigerweise ein Gewichtsverhältnis III:II von 2:1 bis 6:1 eingehalten, insbesondere etwa 3:1. Die Reaktion erfolgt gegebenenfalls in Gegenwart eines inerten Lösungs- oder Verdünnungsmittels, zweckmäßig unter Zusatz einer anorganischen oder organischen Base und gegebenenfalls unter Zusatz eines Reaktionsbeschleunigers. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, Isopropanol, n-Butanol oder Glykol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium- oder Kaliumcarbonat oder Natrium- oder Kaliumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quaternäre Ammoniumsalze wie Tetrabutylammoniumchlorid, -bromid oder -iodid oder Kronenether wie 12-Krone-4, 15-Krone-5, 18-Krone-6 oder Dicyclohexano-18-Krone-6 in Frage.

Die Umsetzung wird im allgemeinen bei Temperaturen zwischen 10 und 15O°C, insbesondere zwischen 20 und 120°C, drucklos oder unter Druck, kontinuierlich oder diskontinuierlich durchgeführt.

Steht Me für ein Metallatom, so wird ein Gewichtsverhältnis III:II von 1:1 bis 3:1, insbesondere 1:1 bevorzugt. Die Reaktion erfolgt gegebenenfalls in Gegenwart eines Lösungs- oder Verdünnungsmittels und gegebenenfalls unter Zusatz einer anorganischen oder organischen Base. Zu den bevorzugten Lösungs- und Verdünnungsmitteln gehören Amide wie Dimethylformamid, Diethylformamid, Dimethylacetamid, N-Methylpyrrolidon, Hexamethylphosphortriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium und Kaliumhydrid, Alkaliamide wie Natrium- und Kaliumamid, ferner Natrium- oder Kalium-tert.-butylat.

Man arbeitet im allgemeinen zwischen -10 und 120°C, vorzugsweise bei 20 bis 80°C. Bei Anwesenheit eines Lösungsmittels wird zweckmäßig beim Siedepunkt des jeweiligen Lösungsmittels gearbeitet.

Die Verbindungen II lassen sich nach bekannten Methoden in einfacher Weise aus den Ketonen der Formel IV 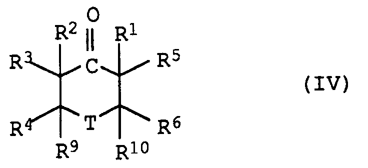
herstellen, z.B. durch Umsetzung mit Trimethylsulfoniummethylsulfat (vgl. Corey, Chaykovsky, J. Am. Chem. Soc. 1962, 64, 3782).

Die Verbindungen IV lassen sich entsprechend allgemein bekannten Verfahren herstellen. Für den Fall, dar R¹ und R⁵ eine >CH-Z-Gruppe bedeuten, ist Cantacuzene, Tordeux, Can. J. Chem. 1976, 54 (17), 2659-2766 zu nennen; bedeuten R¹ und R⁵ eine >C=CH-Gruppe, sei auf Houben-Weyl-Müller, Methoden der organischen Chemie, Georg-Thieme Verlag, Stuttgart, 1972, Bd. V1b verwiesen.

Die Verbindungen der allgemeinen Formel I sowie ihre Salze und Metallkomplexe eignen sich als Fungizide bei guter Pflanzenverträglichkeit.
Herstellungsbeispiele

### Vorschrift A1: 2-(4-Chlorphenoxy)-cyclohexanon

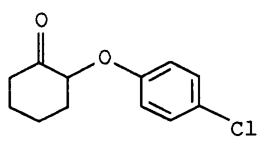

Zu einer Lösung von 5,4 g (0,225 mol) Natriumhydrid (50 %ige Dispersion in Mineralöl) in 100 ml N,N-Dimethylformamid wurden 23,3 g (0,181 mol) 4-Chlorphenol gegeben. Anschließend wurden 20 g (0,151 mol) 2-Chlorcyclohexanon zugesetzt und 24 Stunden bei Raumtemperatur gerührt. Nach Zugabe von 100 ml Wasser und mehrmaliger Extraktion mit Methyl-tert.-butylether wurde die organische Phase mit Wasser gewaschen, über Natriumsulfat getrocknet und im Vakuum eingeengt.
Ausbeute: 32,9 g (97 %)
Schmelzpunkt: 102 bis 104°C.

### Vorschrift B1: 3-(4-Chlorphenoxy)-1-oxa-bicyclo[0,2,5]octan

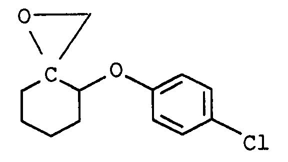

Zu einer Lösung von 12,3 g (0,055 mol) 2-(4-Chlorphenoxy)-cyclohexanon in 100 ml Methylenchlorid wurden 22,7 g (0,121 mol) Trimethylsulfoniummethylsulfat und 30 g Natronlauge (konz.) gegeben. Nachdem das Reaktionsgemisch 12 bis 15 Stunden bei Raumtemperatur (20°C) gerührt worden war, wurden der Lösung 100 ml Wasser zugesetzt und die organische Phase abgetrennt. Die organische Phase wurde zweimal mit Wasser gewaschen, über Natriumsulfat getrocknet und eingeengt.
Ausbeute: 11,5 g (88 %).

### Vorschrift B2: 3-(4-Chlorbenzyliden)-1-oxa-bicyclo(0,2,5]octan

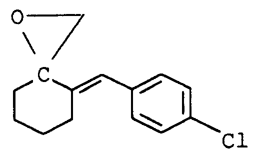

Es wurde analog Beispiel B1 gearbeitet, wobei 23,5 g (0,108 mol) 2-(4-Chlorbenzyliden)-cyclohexanon in 100 ml Methylenchlorid, 24 g (0,128 mol) Trimethylsulfoniummethylsulfat und 50 ml Natronlauge (50 gew.-%ig) verwendet wurden.
Ausbeute: 24 g (95 %)

### Beispiel 1

### 1-(1,2,4-Triazol-1-yl-methyl)-2-(4-chlorphenoxy)-cyclohexan-1-ol (nicht erfindungsgemäß)

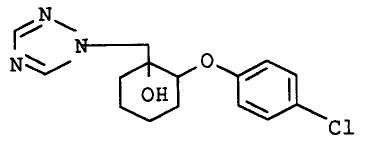

Eine Lösung von 3,5 g (0,051 mol) 1,2,4-Triazol in 50 ml N,N-Dimethylformamid wurde mit 3,4 g Natronlauge (50 gew.-%ig) versetzt und für 30 Minuten auf 50°C erwärmt. Anschließend wurden bei Raumtemperatur 8,0 g (0,033 mol) 3-(4-Chlorphenoxy)-1-oxa-bicyclo[0,2,5]octan, das in 20 ml N,N-Dimethylformamid gelöst war, zugetropft. Nachdem das Reaktionsgemisch 15 Stunden bei Raumtemperatur gerührt worden war, wurden der Lösung 100 ml Wasser zugesetzt und mehrmals mit Methyl-tert.-butylether ausgeschüttelt. Die isolierte organische Phase wurde zweimal mit Wasser gewaschen, daraufhin über Natriumsulfat getrocknet und eingeengt. Durch Kristallisation des Rückstandes aus Methyl-tert.-butylether/n-Hexan erhielt man das Produkt als 2:1 Diastereomerengemisch.
Ausbeute: 6,5 g (63 %)
Schmelzpunkt: 120 bis 122°C.

### Beispiel 2

### 1-(1, 2, 4-Triazol-1-yl-methyl)-2-(4-chlorphenyliden)-cyclohexan-1-ol (nicht erfindungsgemäß)

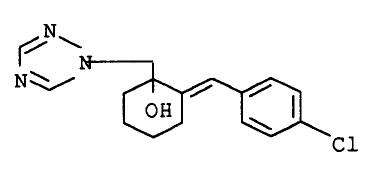

Es wurde analog Beispiel C1 eine Lösung von 6,2 g (0,091 mol) 1,2,4-Triazol in 100 ml N,N-Dimethylformamid mit 6,8 g Natronlauge (50 gew.-%ig) versetzt und für 30 Minuten auf 50°C erwärmt. Nachdem das Reaktionsgemisch auf Raumtemperatur gekühlt worden war, wurden der Lösung 10,5 g (0,045 mol) 3-(4-Chlorbenzyliden)-1-oxa-bicyclo[0,2,5]octan, das in 50 ml N,N-Dimethylformamid gelöst war, zugetropft und 12 Stunden bei Raumtemperatur gerührt. Anschließend wurden 100 ml Wasser zugegeben und mehrmals mit Methyl-tert.-butylether extrahiert und die organische Phase mit Wasser gewaschen, über Natriumsulfat getrocknet und im Vakuum eingeengt.
Ausbeute: 10,5 g (77 %).

Entsprechend den Beispielen 1 bzw. 2 können die in den Tabellen 1 bzw. 2 aufgeführten Verbindungen hergestellt werden.

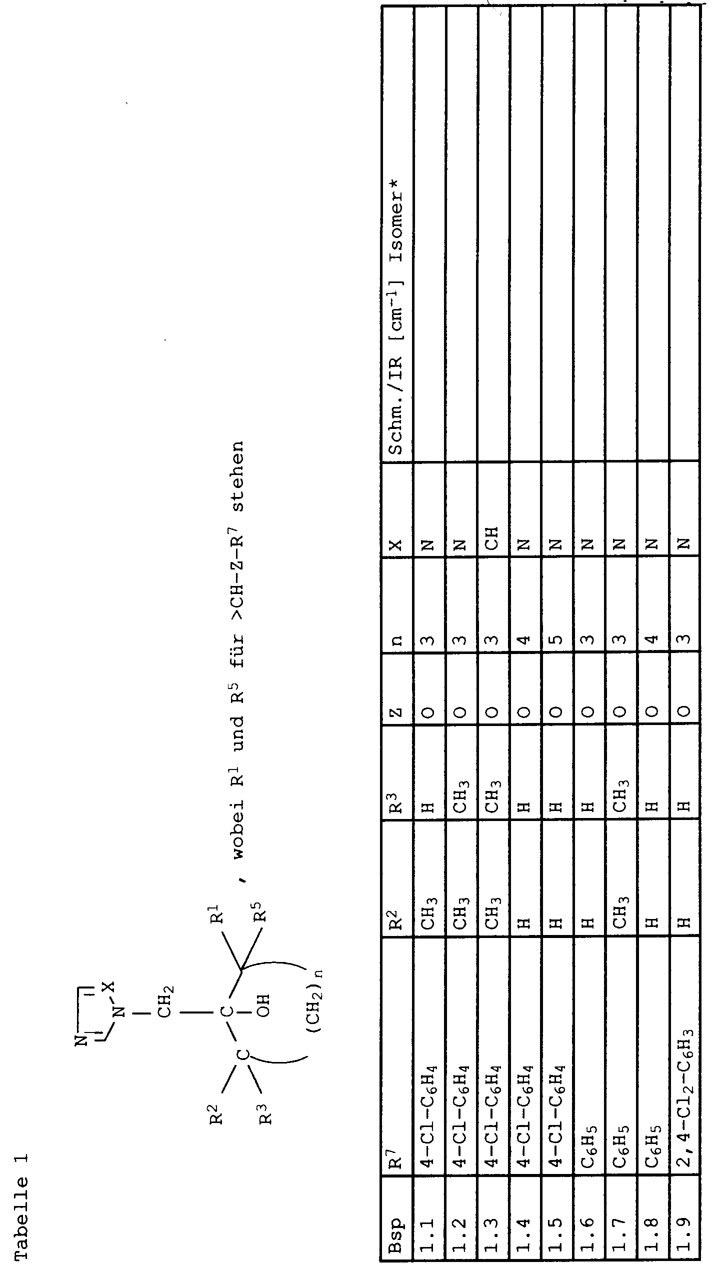

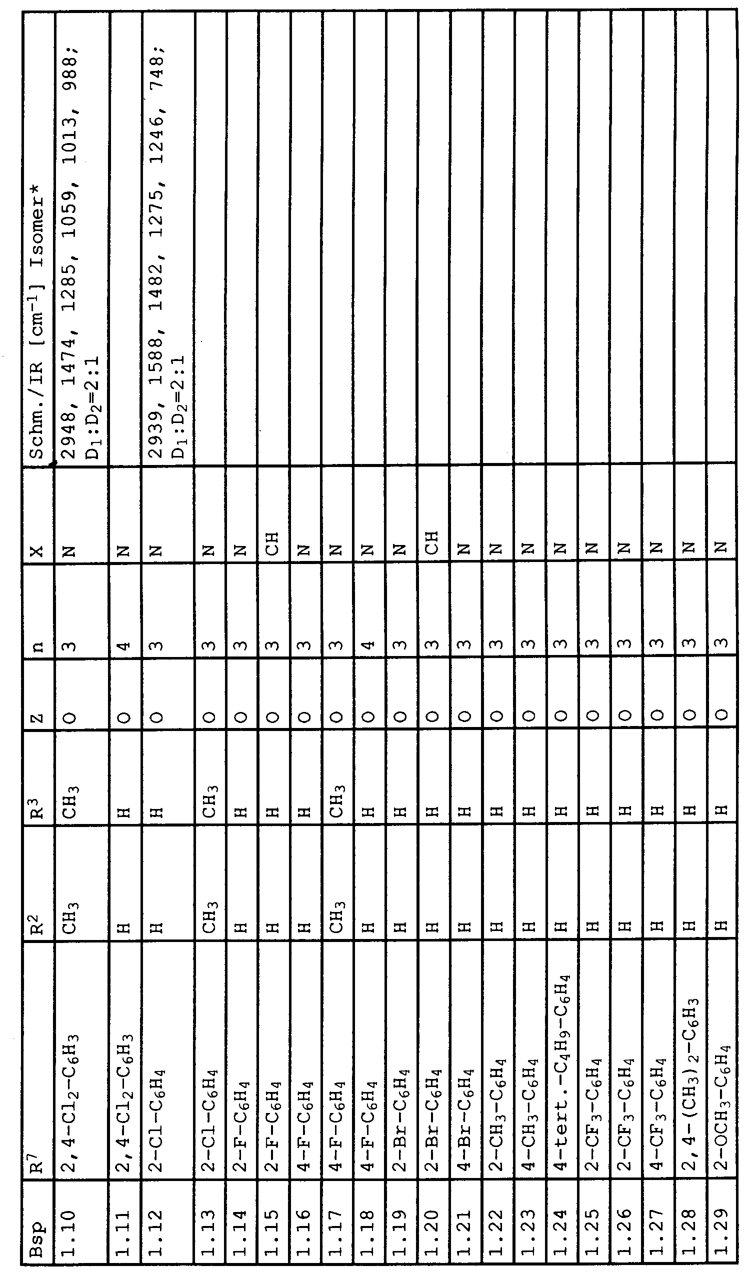

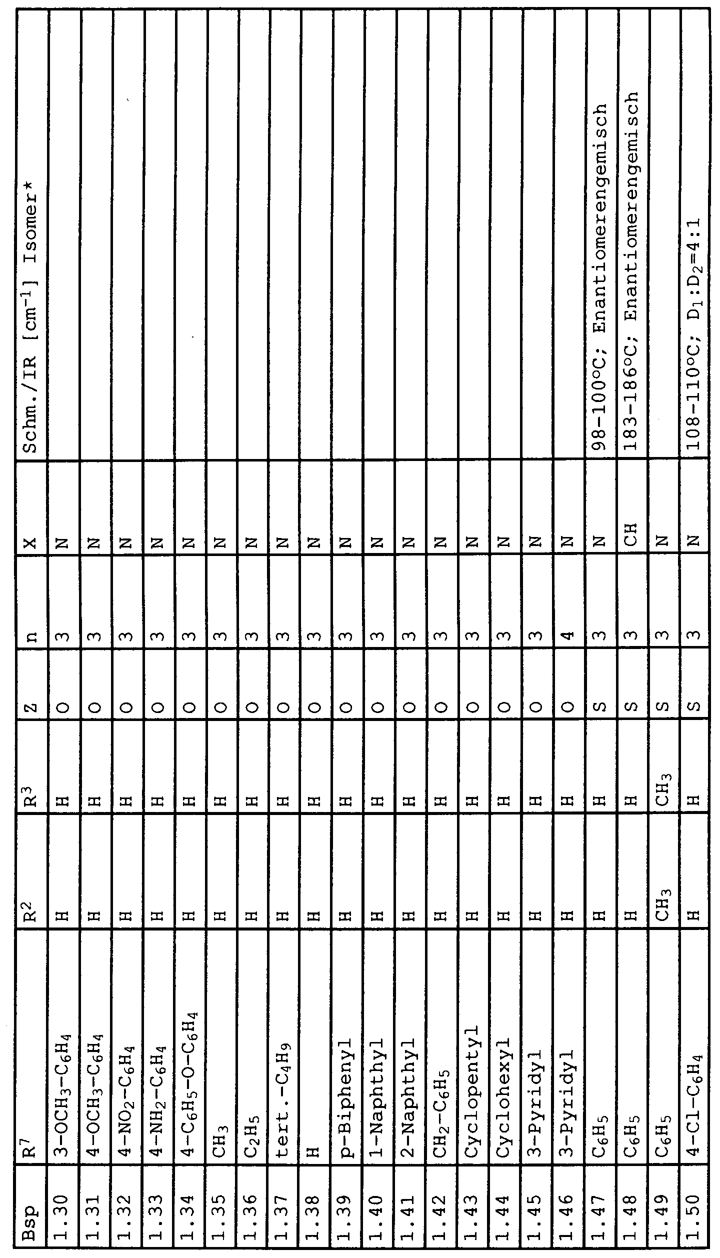

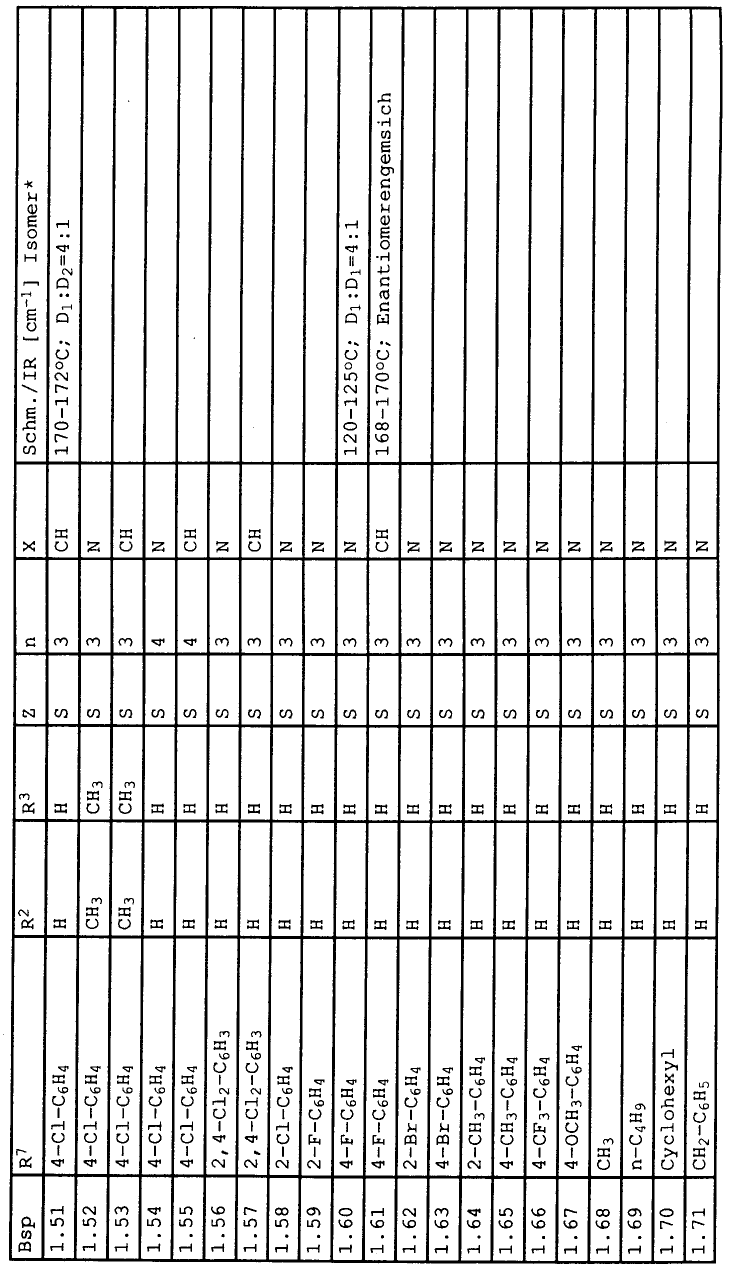

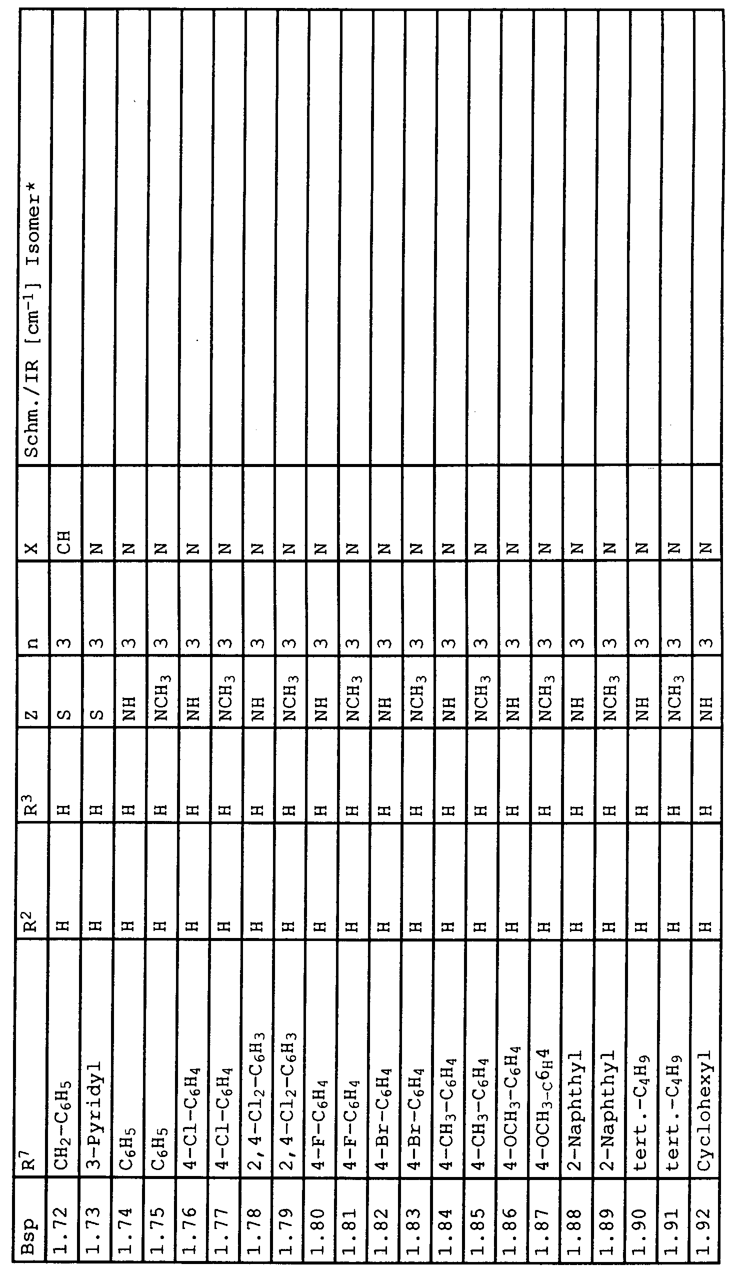

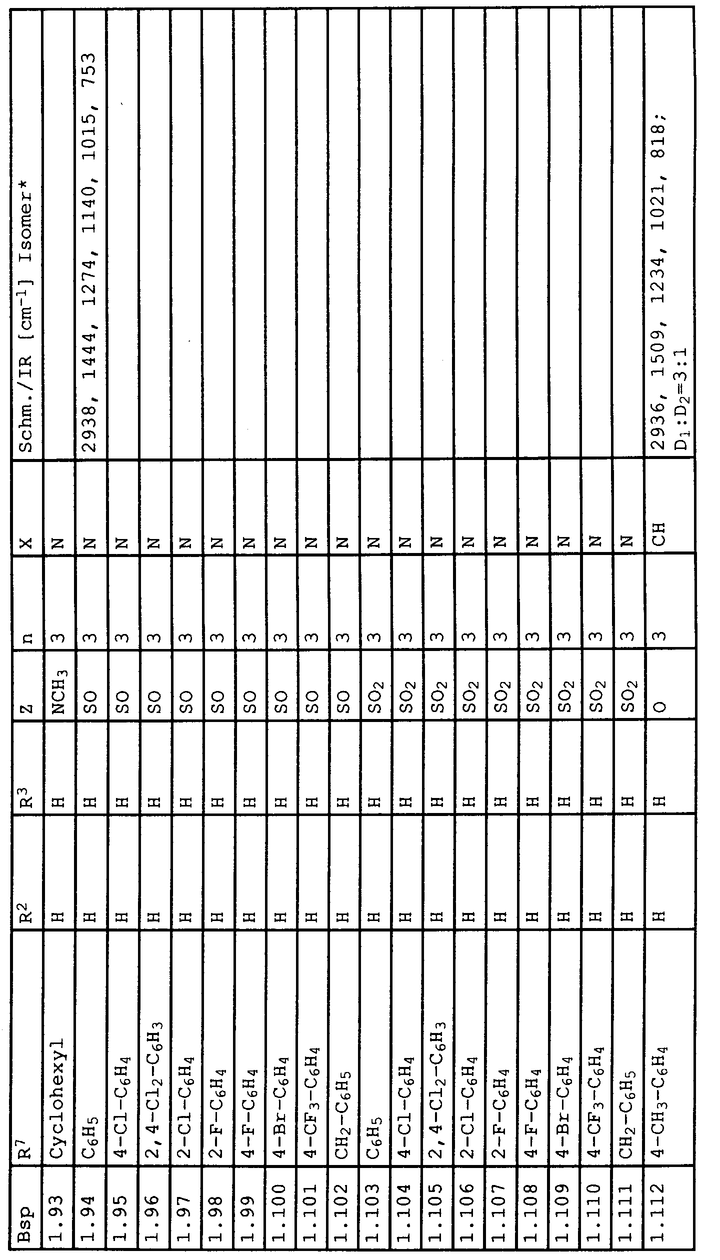

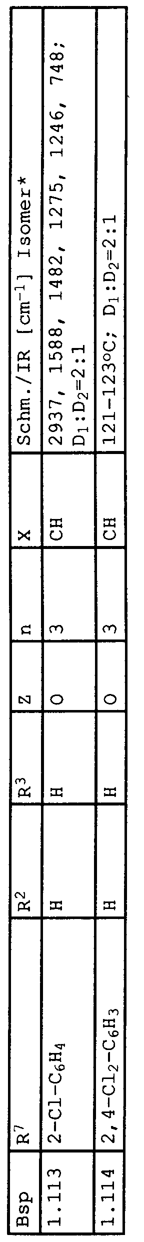

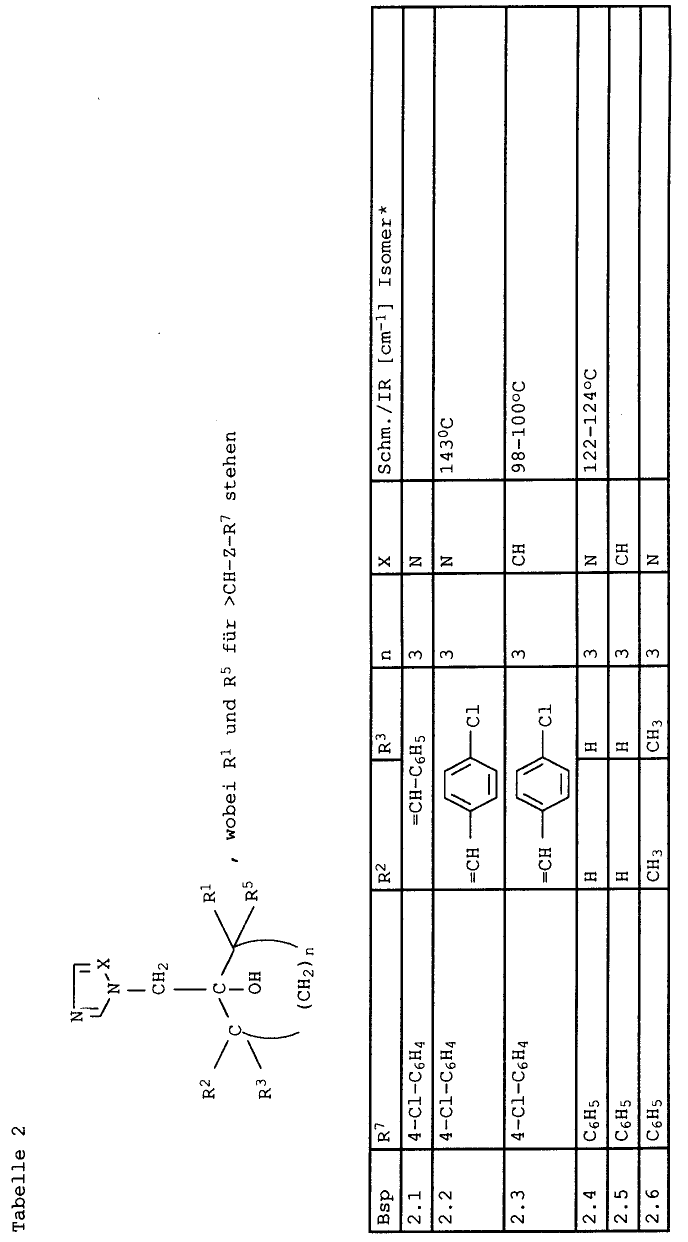

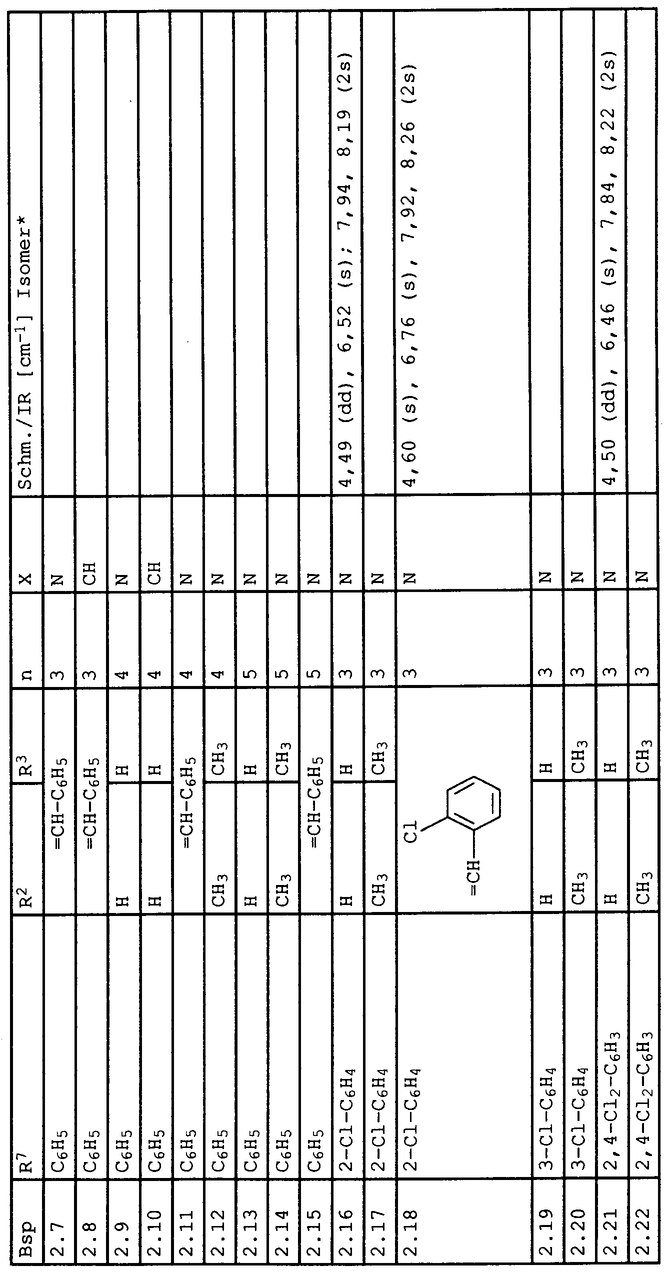

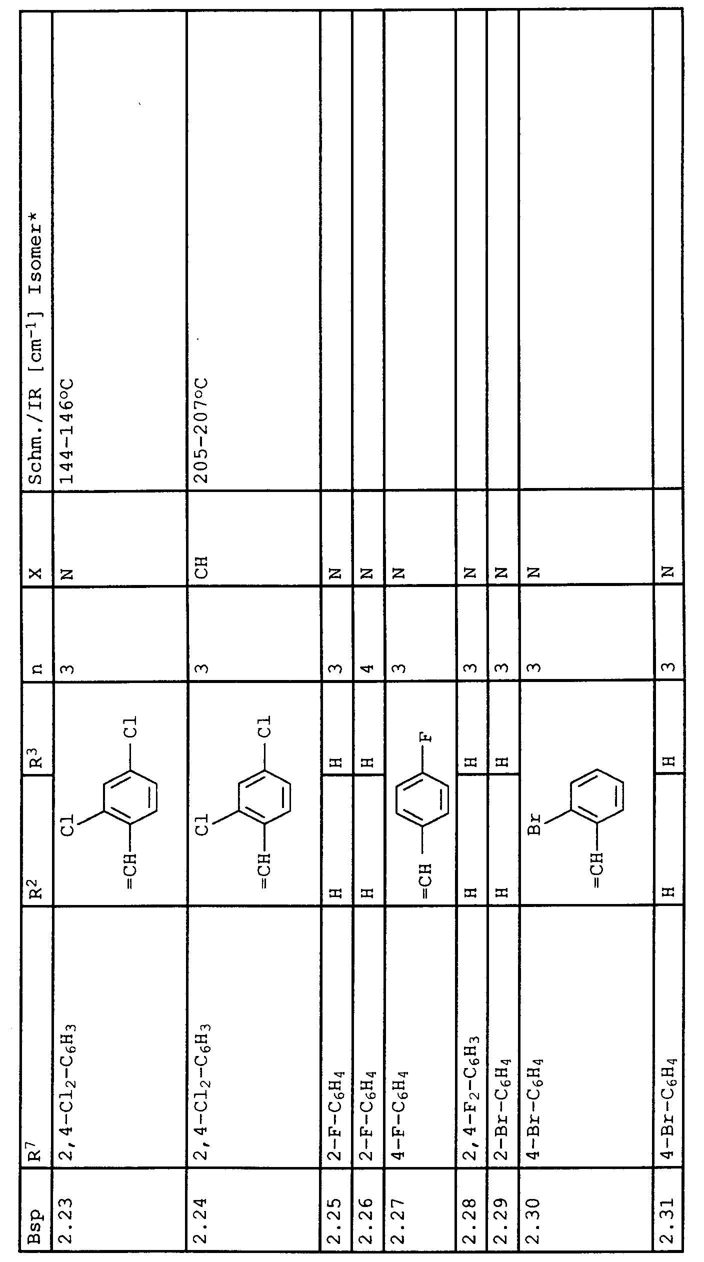

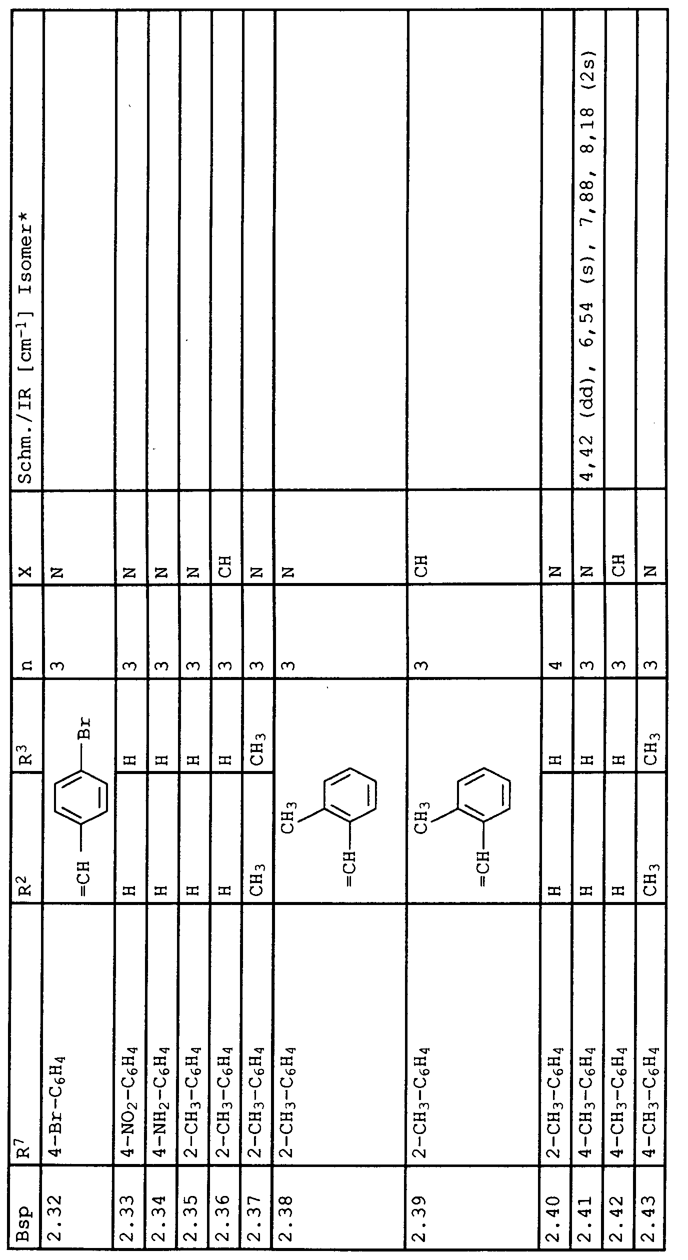

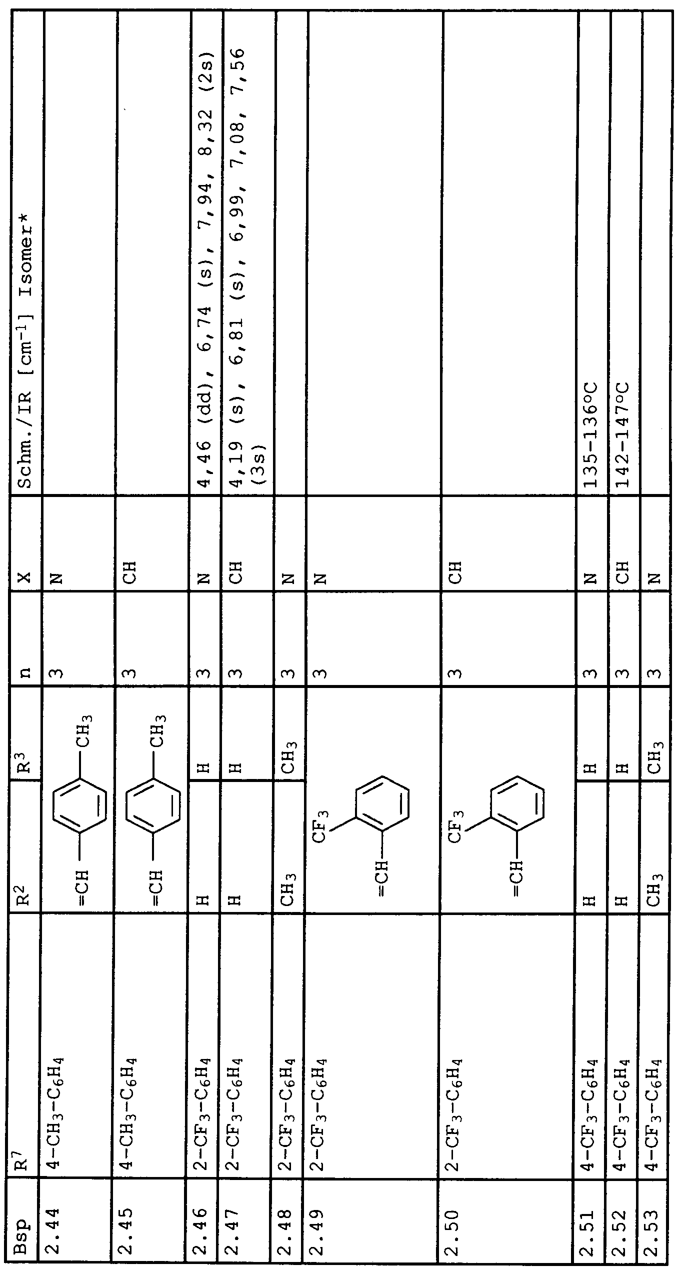

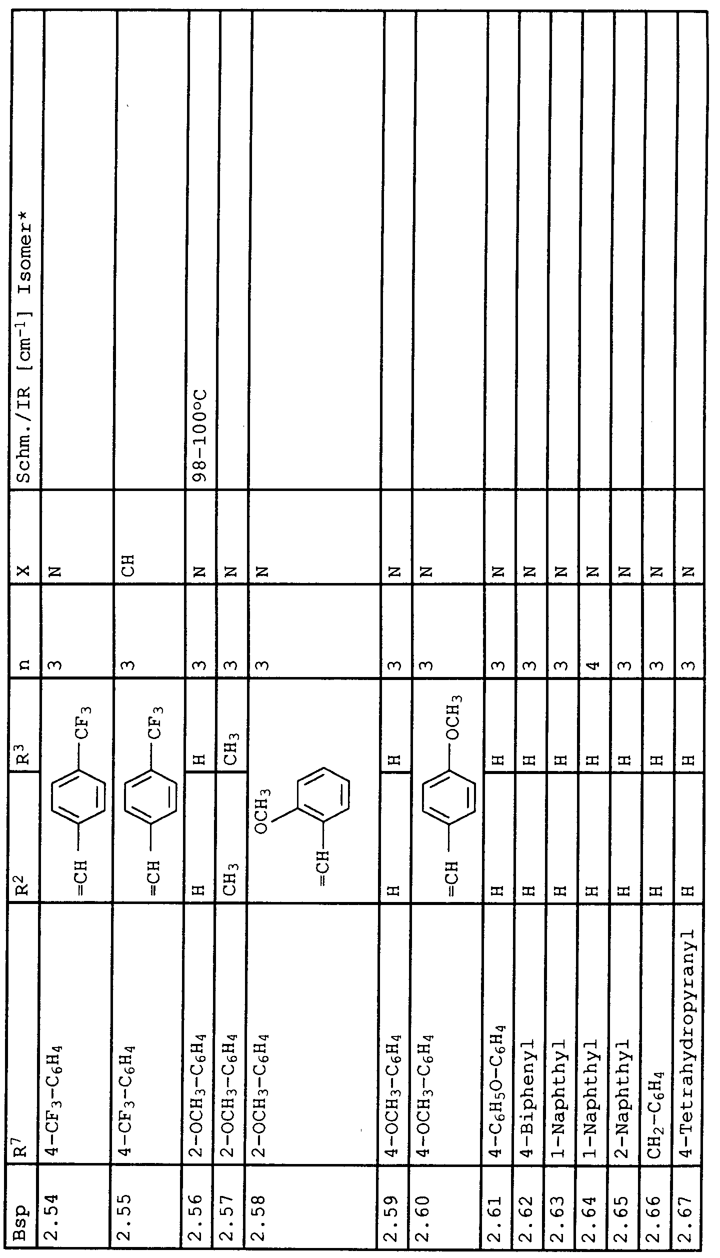

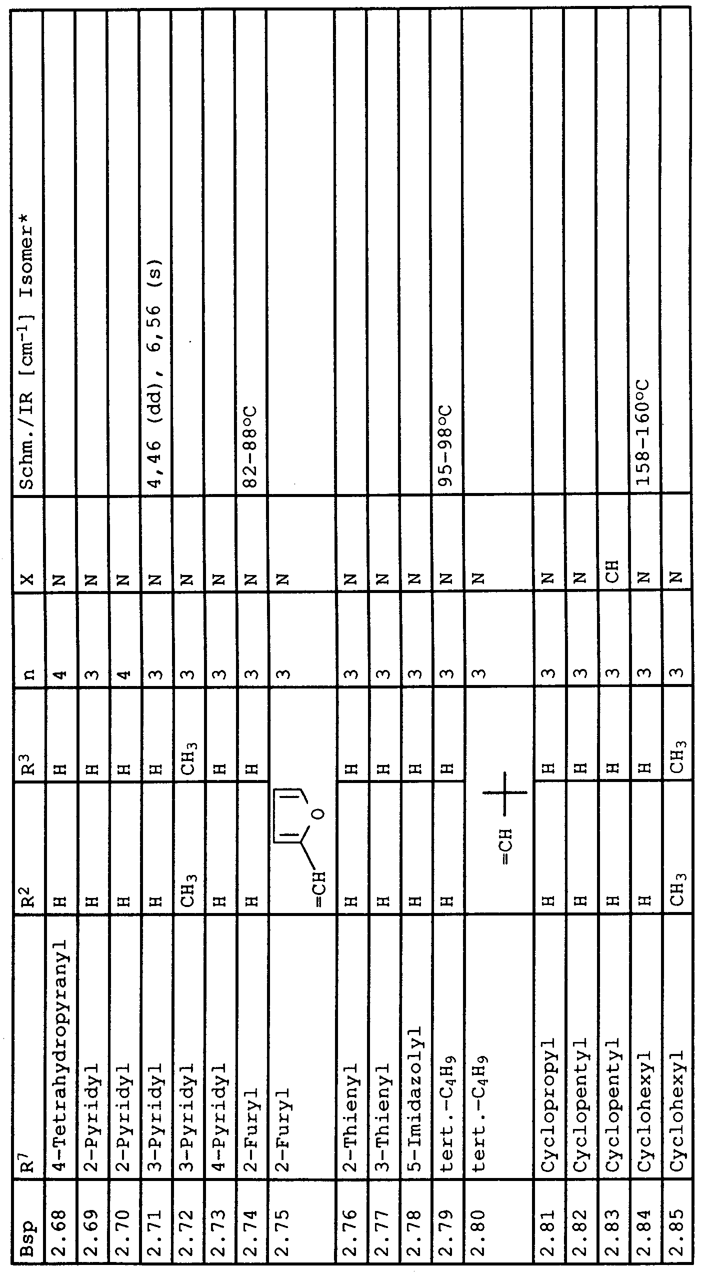

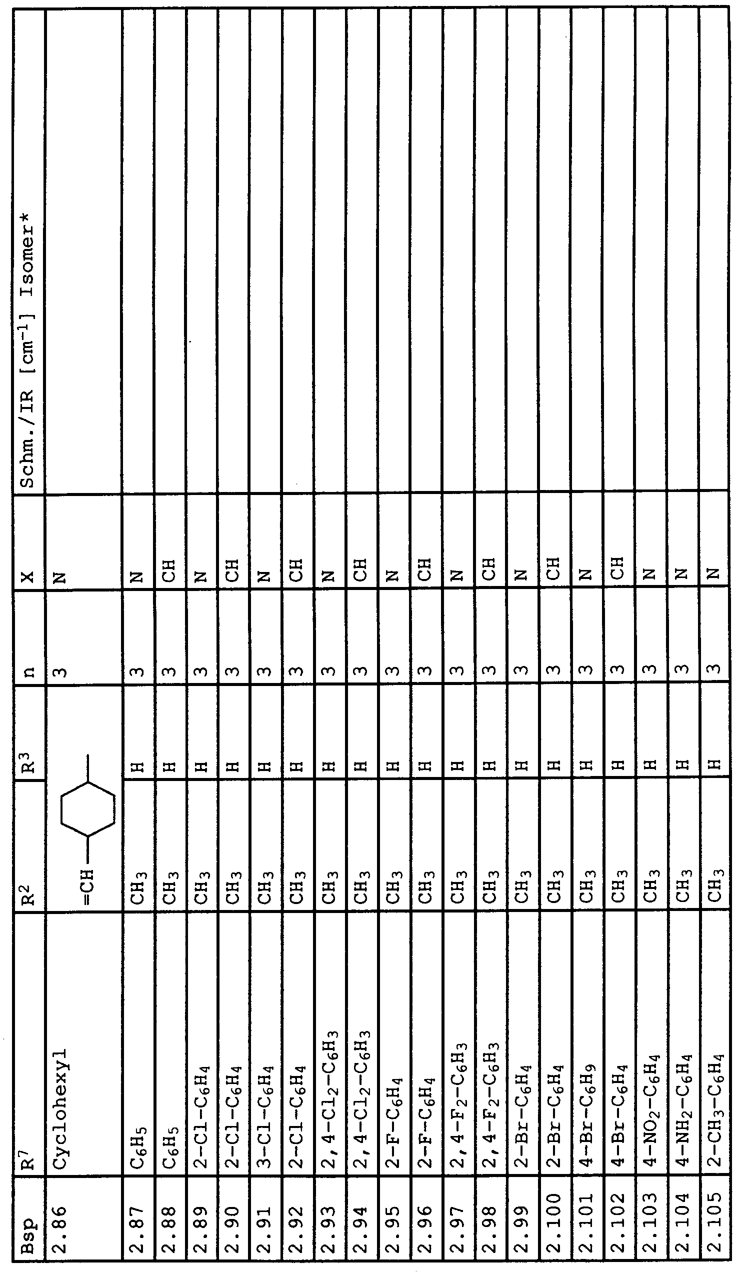

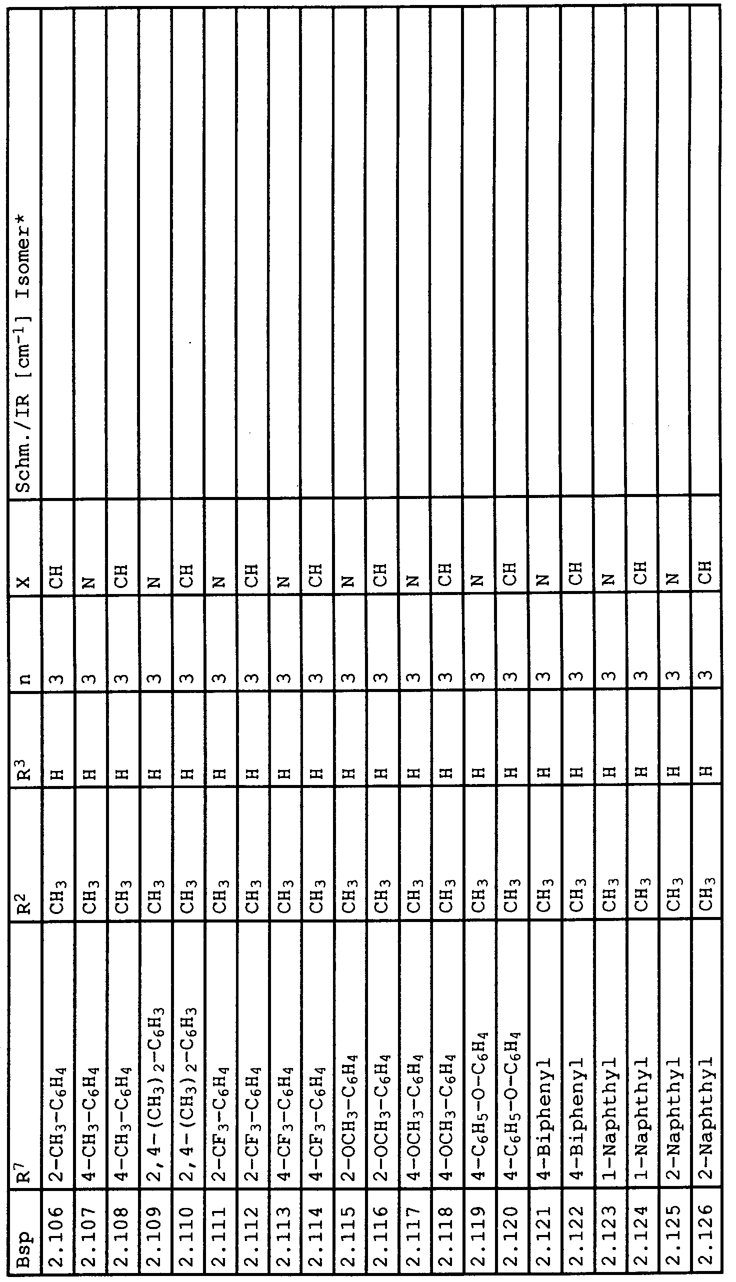

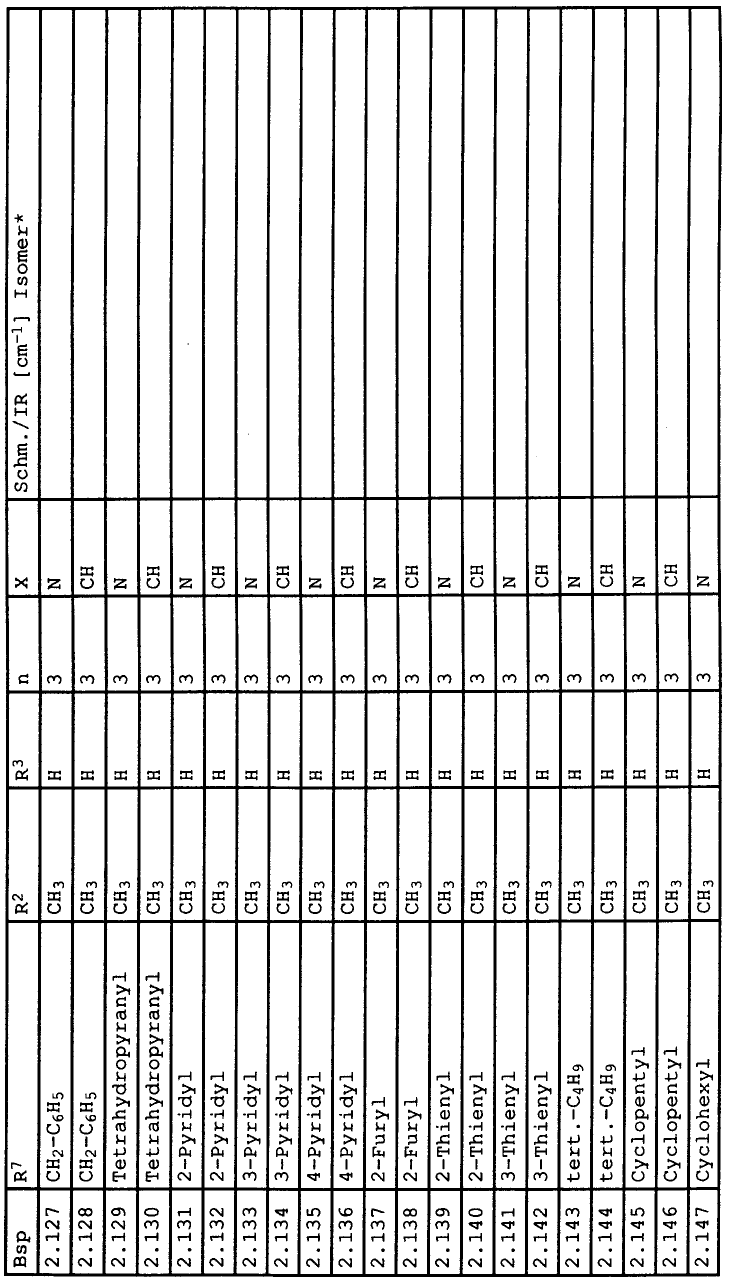

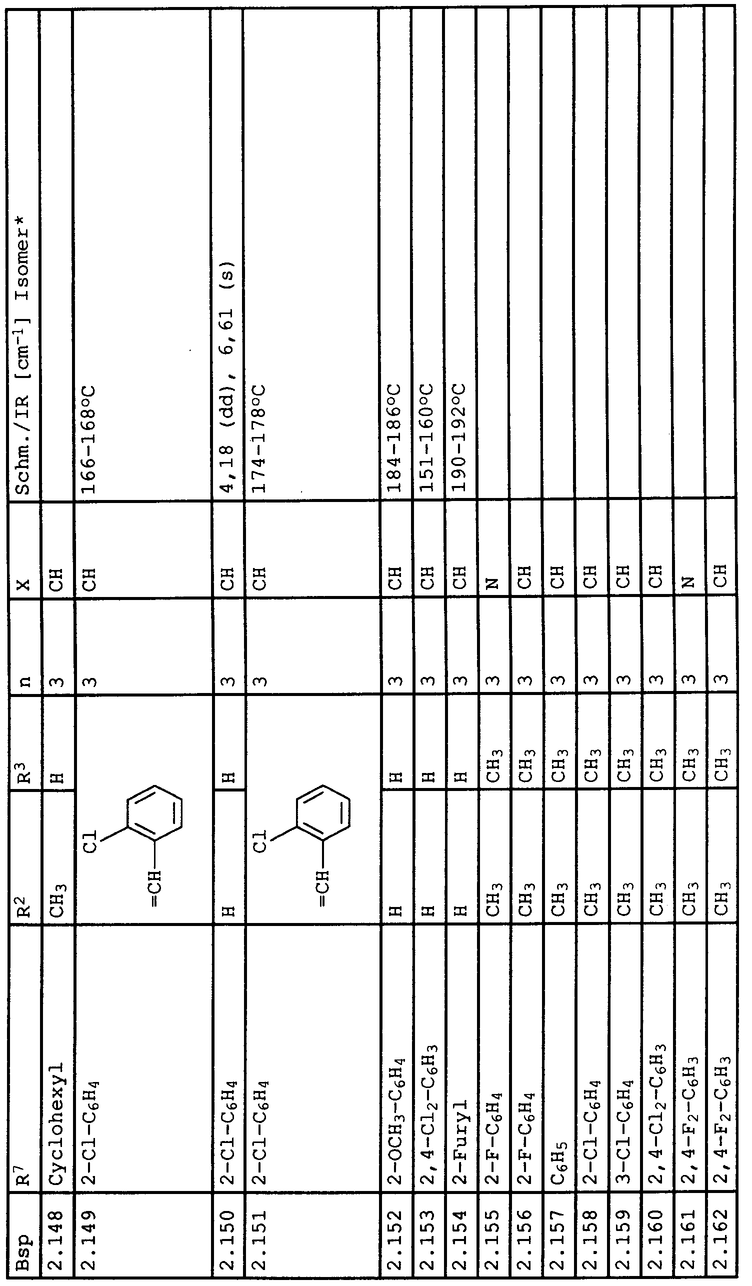

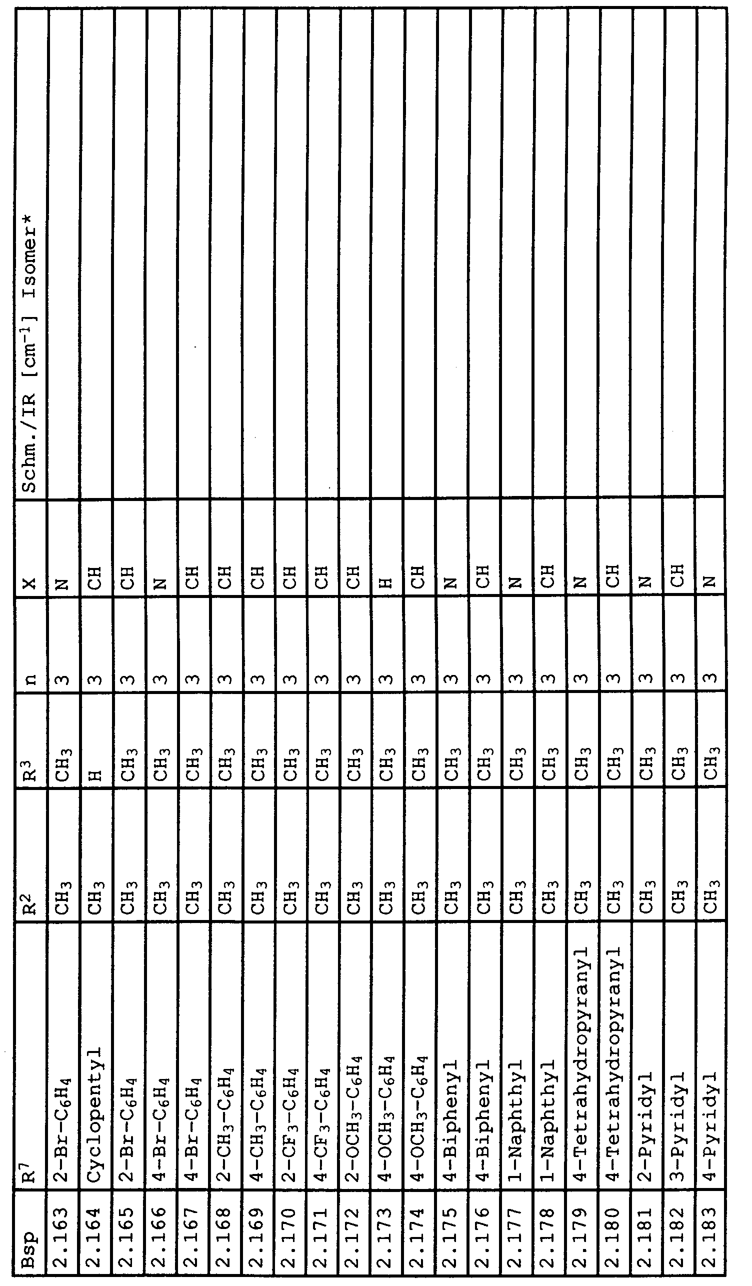

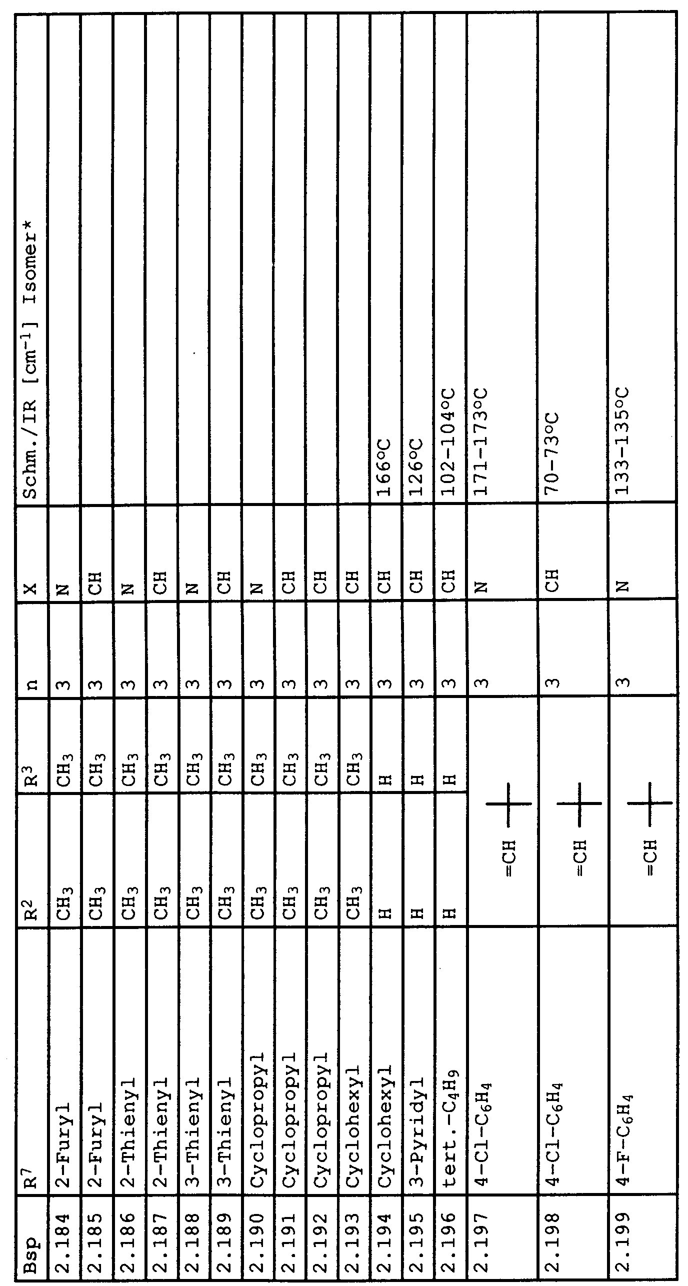

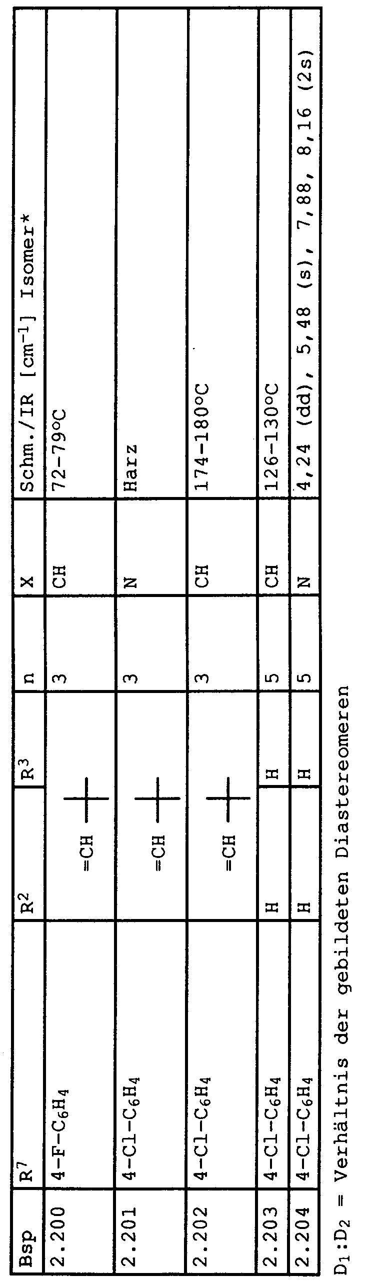

### Vorschrift B3

### 2-(4-Chlorbenzyliden)-spiro-(trans-decalin-1,2-oxiran)

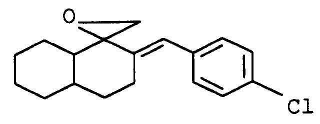

Zu einer Lösung von 30,2 g (0,11 mol) 2-(4-Chlorbenzyliden)-decalon (1) in 200 ml Methylenchlorid wurden 45,5 g (0,24 mol) Trimethylsulfoniummethylsulfat und 42 ml Natronlauge (konz.) gegeben. Nachdem das Reaktionsgemisch 12 Stunden bei Raumtemperatur (20°C) gerührt worden war, wurden der Lösung 100 ml Wasser zugesetzt und die organische Phase abgetrennt. Die organische Phase wurde zweimal mit Wasser gewaschen, über Natriumsulfat getrocknet und eingeengt.
Ausbeute: 29 g (91 %)
Schmelzpunkt: 102 bis 105°C

### Beispiel 3

### 1-(1,2,4-Triazol-1-ylmethyl)-2-(4-chlorbenzyliden)-decan-1-ol (nicht erfindungsgemäß)

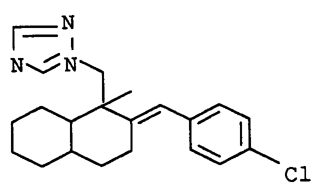

Eine Lösung von 6,9 g (0,1 mol) 1,2,4-Triazol in 50 ml N,N-Dimethylformamid wurde mit 5,2 ml Natronlauge (50 gew.-%ig) versetzt und für 30 Minuten auf 50°C erwärmt. Anschließend wurden bei Raumtemperatur 14,4 g (0,05 mol) 2∼(4∼chlorbenzyliden)∼spiro∼(trans-decalin-1,2-oxiran), das in 30 ml N,N-Dimethylformamid gelöst war, zugetropft. Nachdem das Reaktionsgemisch 15 Stunden bei Raumtemperatur gerührt worden war, wurden der Lösung 100 ml Wasser zugesetzt und mehrmals mit Methyl-tert.-butylether ausgeschüttelt. Die isolierte organische Phase wurde zweimal mit Wasser gewaschen, daraufhin über Natriumsulfat getrocknet und eingeengt. Durch Kristallisation des Rückstandes aus Methyl-tert.butylether/n-Hexan erhielt man das Produkt als Enantiomerengemisch.
Ausbeute: 13,3 g (63 %)
Schmelzpunkt: 180 bis 186°C
Entsprechend Beispiel 3 können die in Tabelle 3 aufgeführten Verbindungen hergestellt werden.

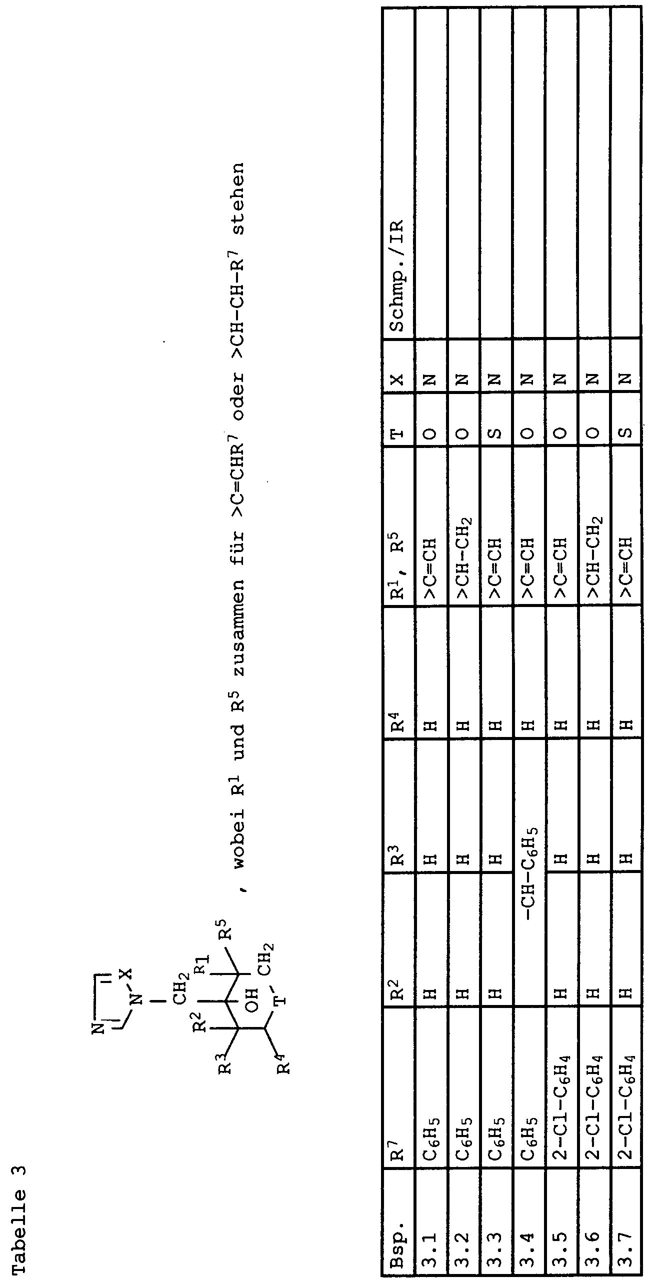

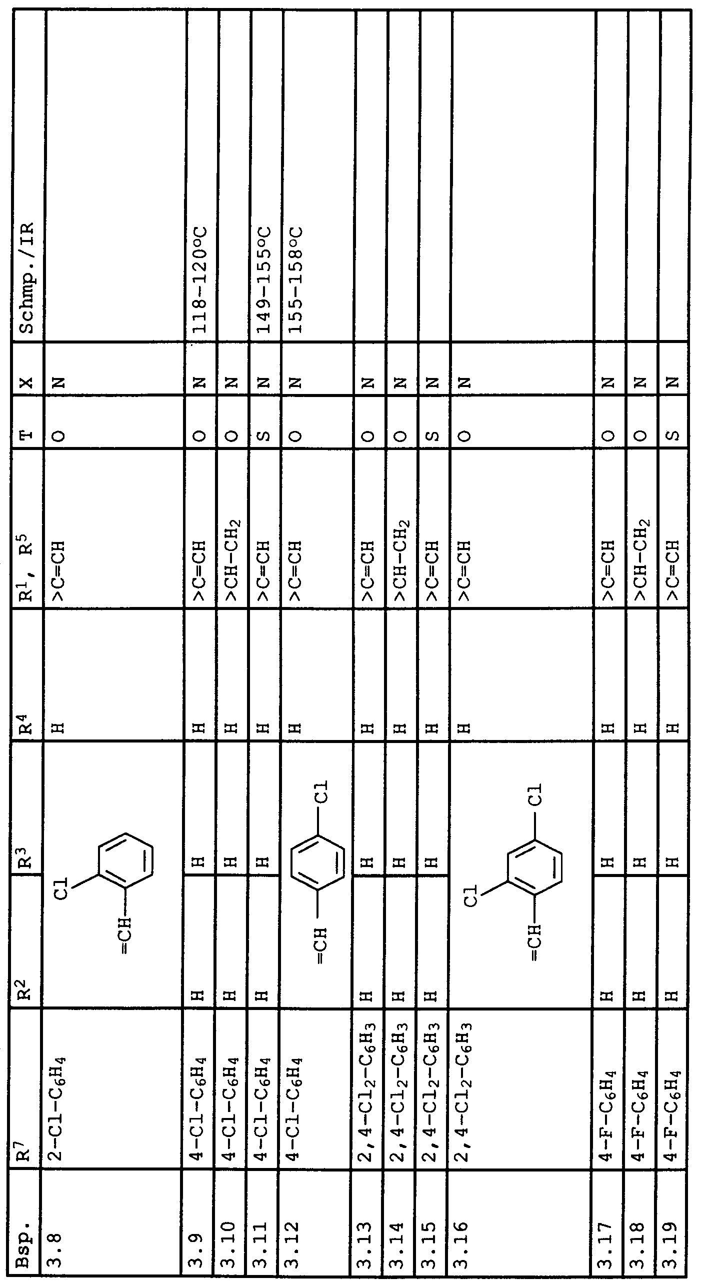

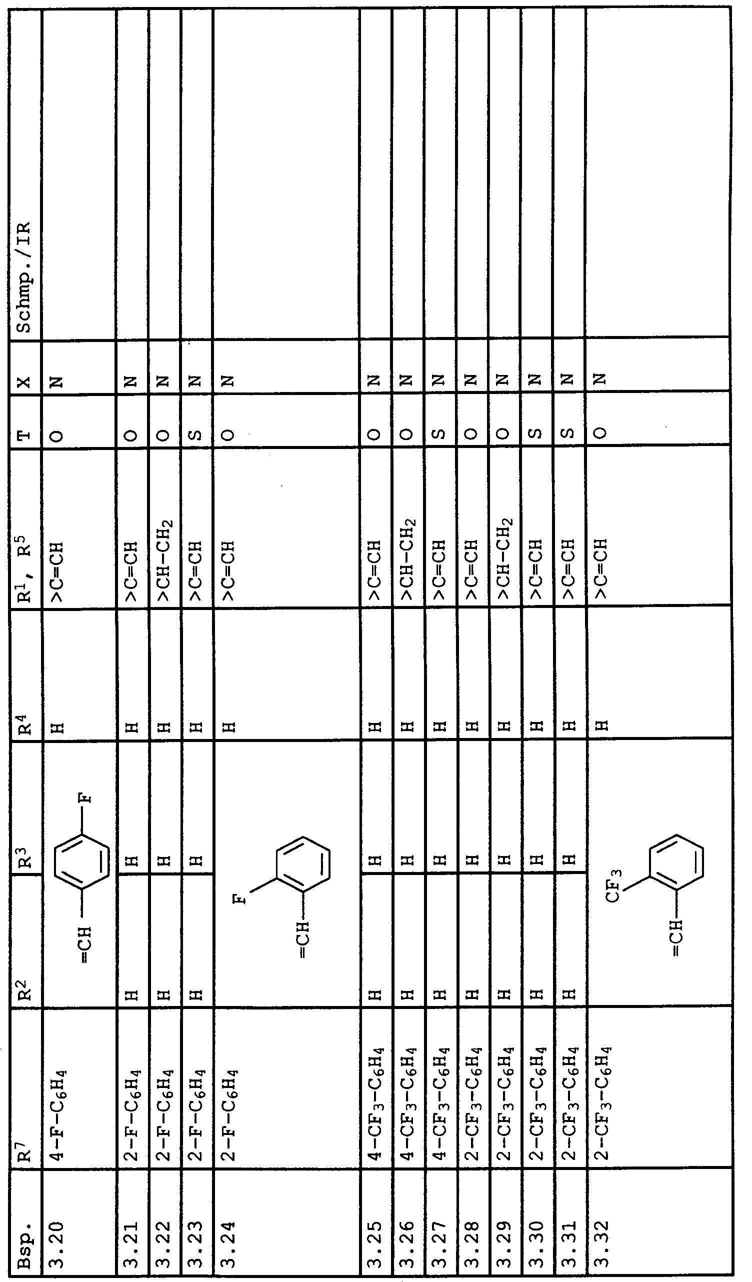

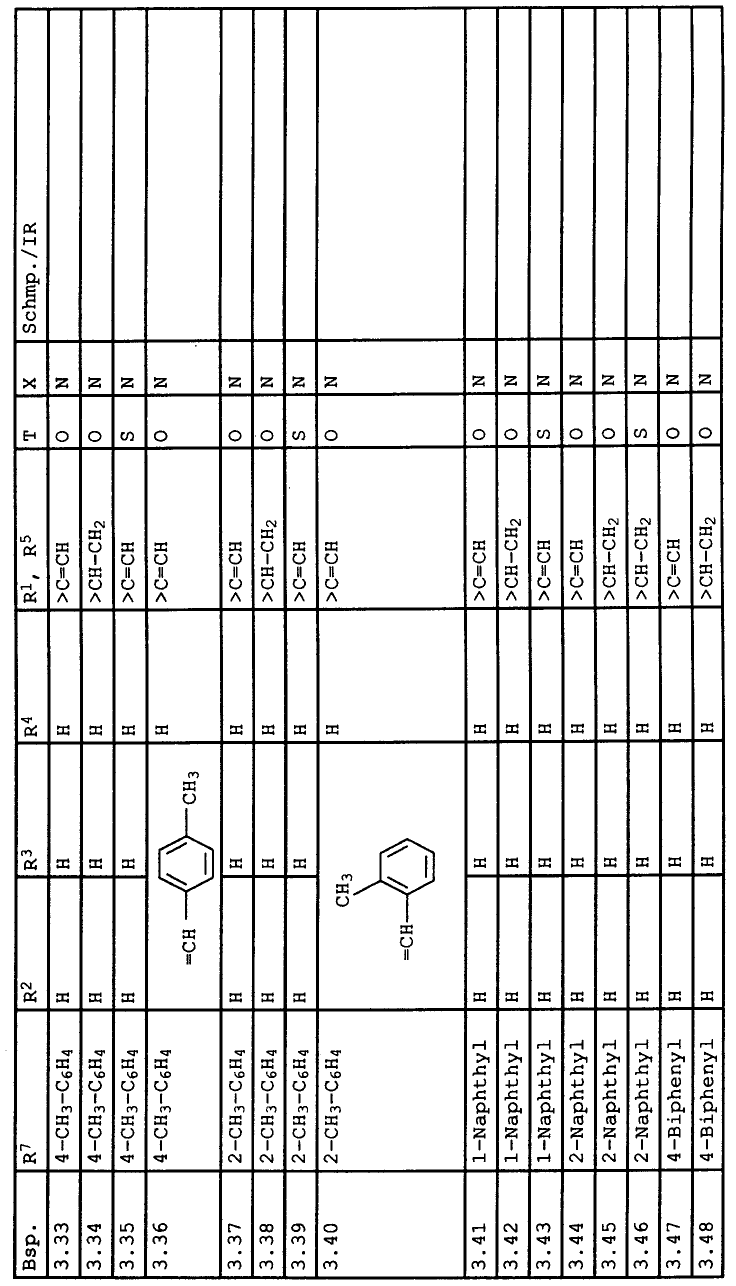

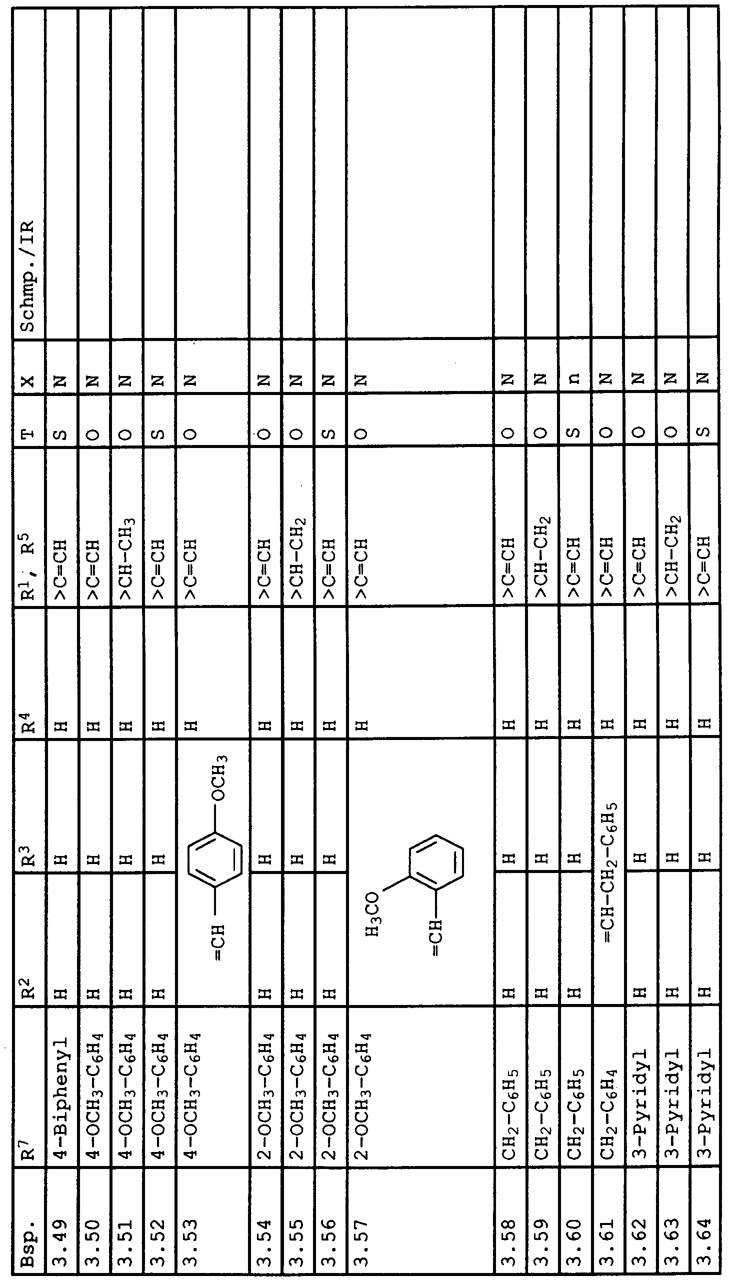

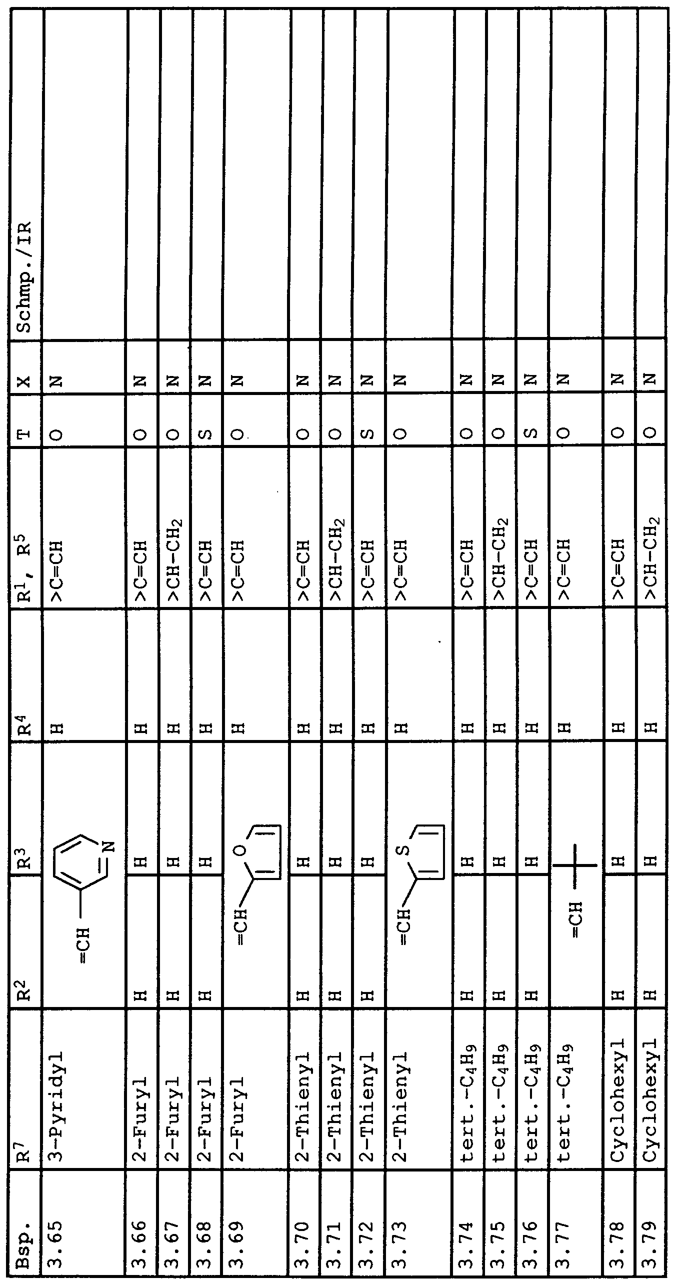

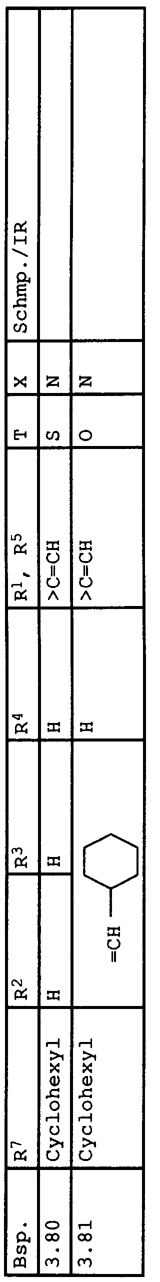

### Vorschrift B4

### 4-(4-Chlorphenyl)-1-oxa-bicyclo(0,2,5)octan

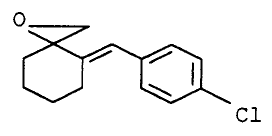

Zu einer Lösung von 30 g (0,14 mol) 3-(4-Chlorphenyl)-cyclohexanon in 100 ml Methylenchlorid wurden 54,4 g (0,30 mol) Trimethylsulfoniummethylsulfat und 50 ml Natronlauge (50 %) gegeben. Nachdem das Reaktionsgemisch 12 Stunden bei Raumtemperatur (20°C) gerührt worden war, wurden der Lösung 100 ml Wasser zugesetzt und die organische Phase abgetrennt. Die organische Phase wurde zweimal mit Wasser gewaschen, über Natriumsulfat getrocknet und eingeengt.
Ausbeute: 28,2 g (91 %)

### Beispiel 4

### 1-(1,2,4-Triazol-1-yl-methyl)-3-(4-chlorphenyl)-cyclohexan-1-ol (Verbindung Nr. 4.1, Tabelle 4)

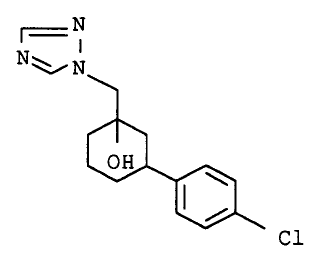

Eine Lösung von 11,3 g (0,16 mol) Triazol in 100 ml N,N-Dimethylformamid wurde mit 12,4 g Natronlauge (50 gew.-%ig) versetzt und für 30 Minuten auf 50°C erwärmt. Anschließend wurden bei Raumtemperatur 18,2 g (0,08 mol) 4-(4-Chlorphenyl)-1-oxa-bicyclo[0,2,5]octan, das in 20 ml N,N-Dimethylformamid gelöst war, zugetropft. Nachdem das Reaktionsgemisch 15 Stunden bei Raumtemperatur gerührt worden war, wurden der Lösung 100 ml Wasser zugesetzt und mehrmals mit Methyl-tert.-butylether ausgeschüttelt. Die organische Phase wurde zweimal mit Wasser gewaschen, über Natriumsulfat getrocknet und eingeengt. Durch Kristallisation des Rückstandes aus Methyltert.-butylether/n-Hexan erhielt man das Produkt als 2:1-Diastereomerengemisch.
Ausbeute: 20,3 g (87 %)
Schmelzpunkt: 128 bis 130°C.

Entsprechend Beispiel 4 können die in der Tabelle 4 aufgeführten Verbindungen hergestellt werden.

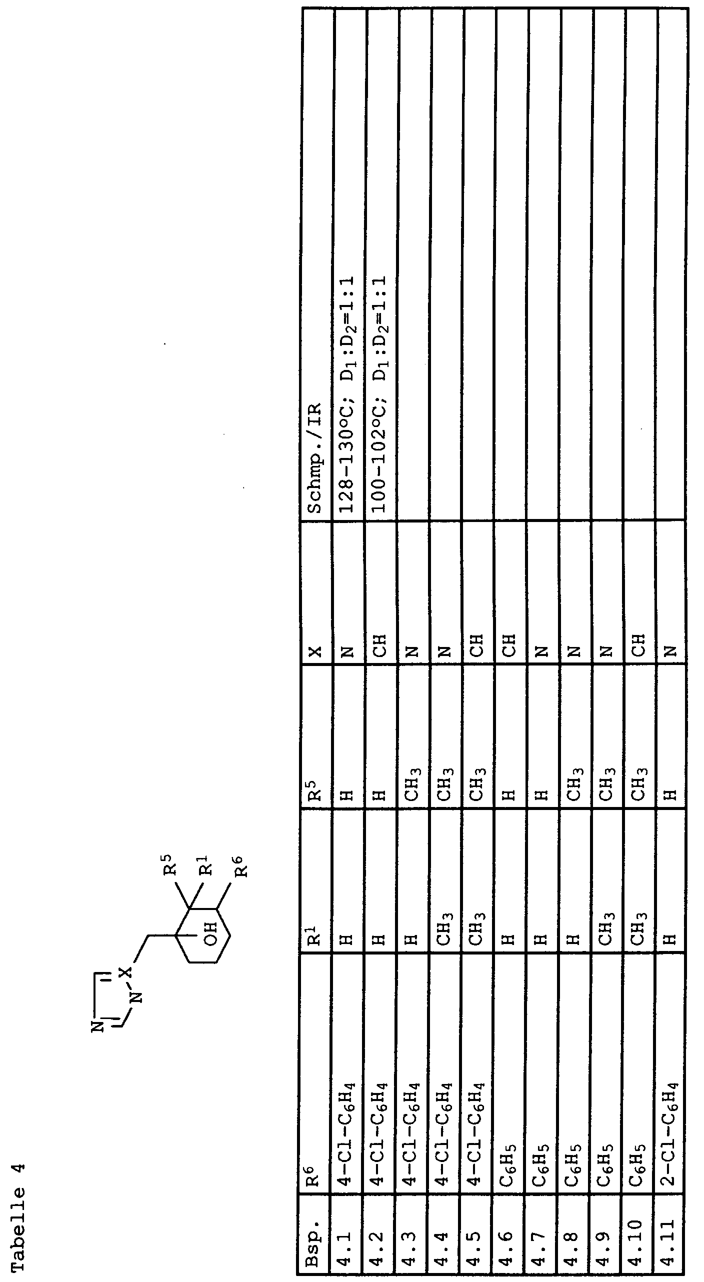

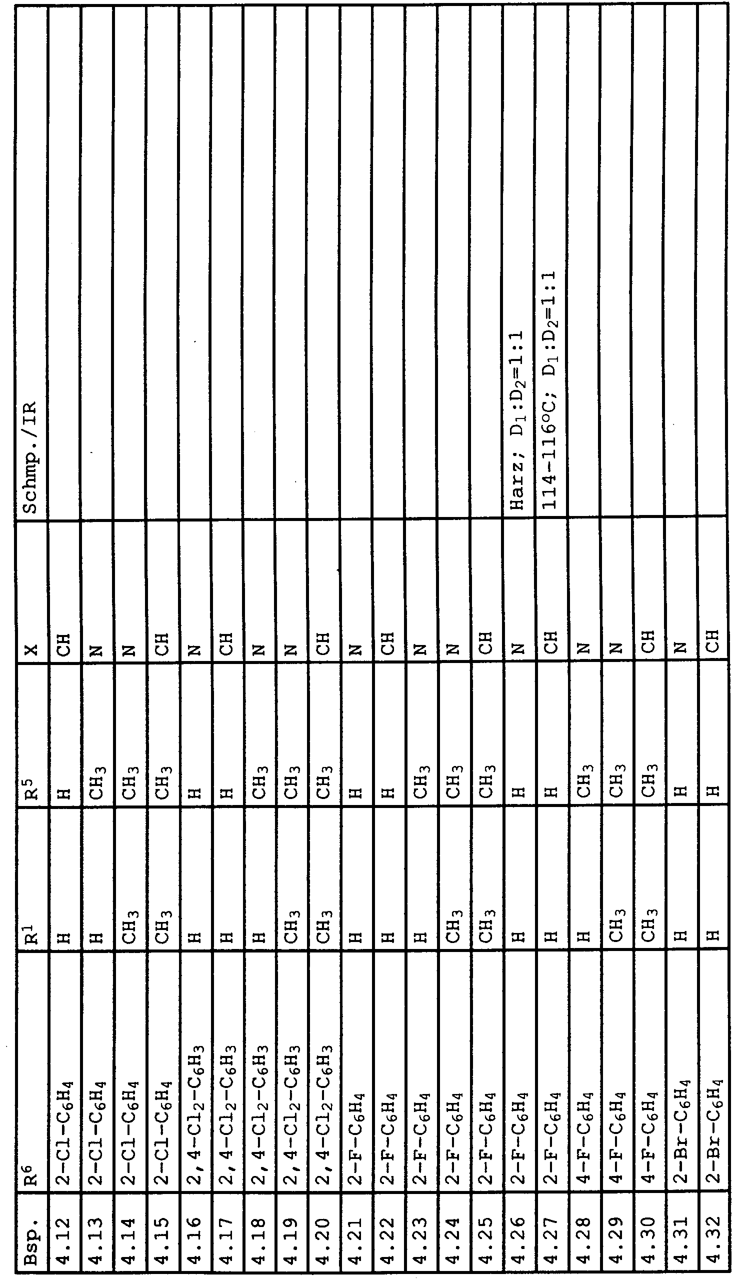

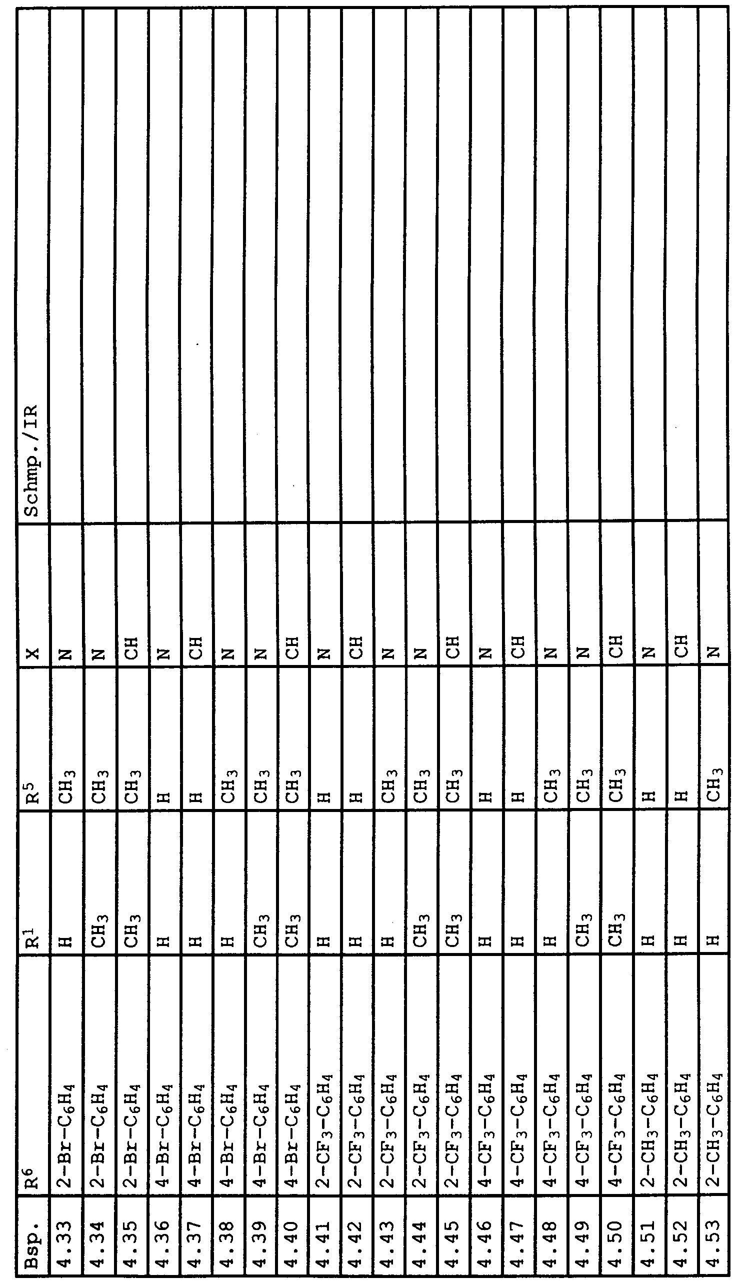

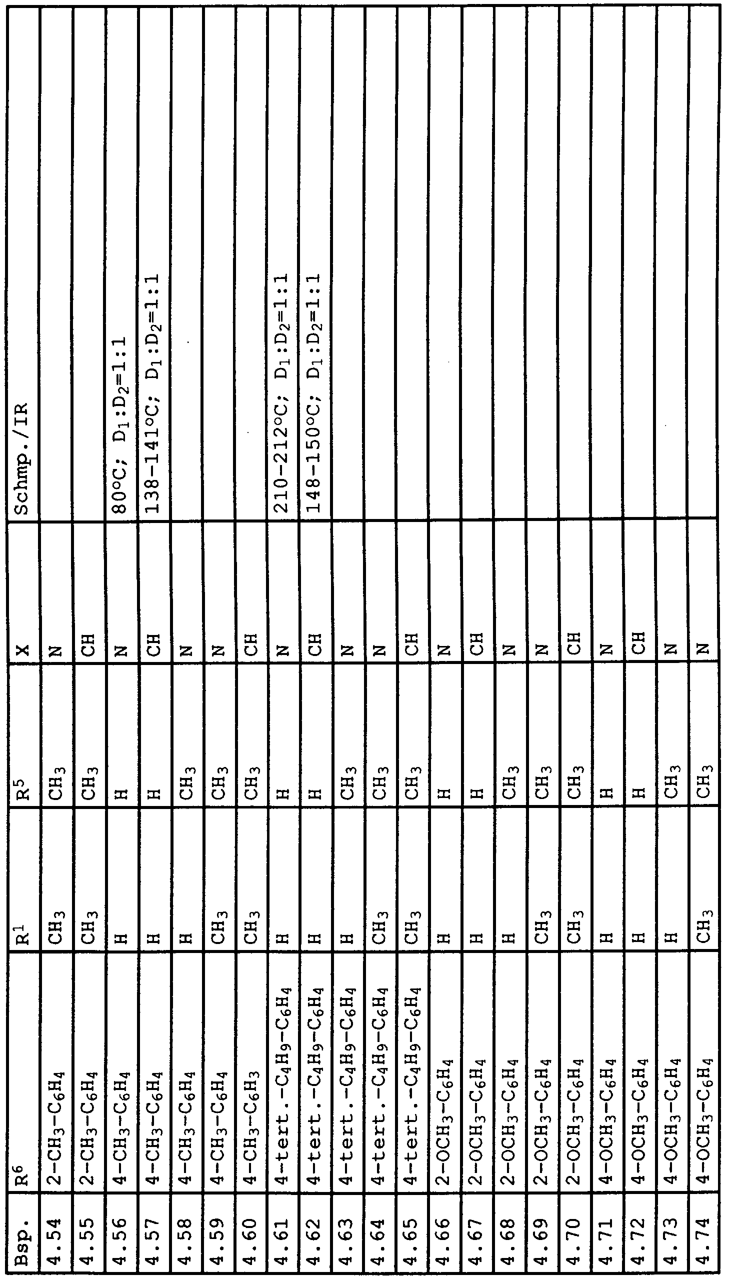

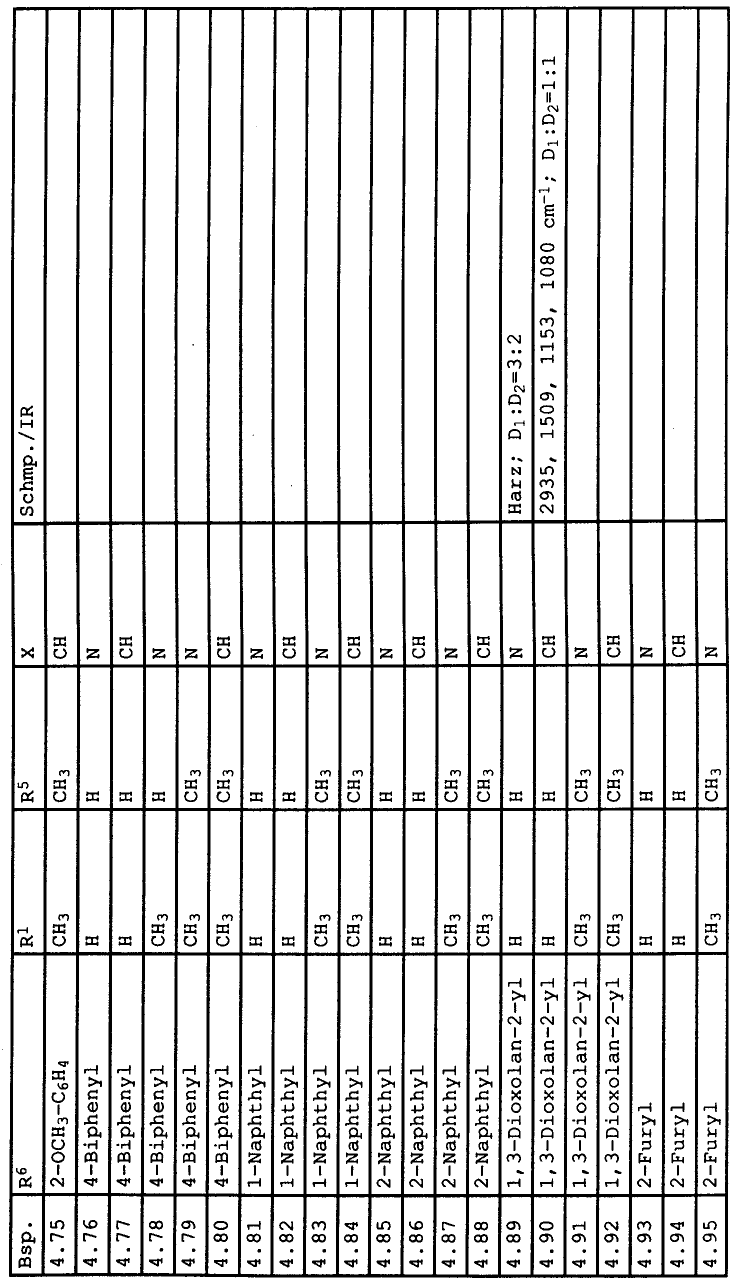

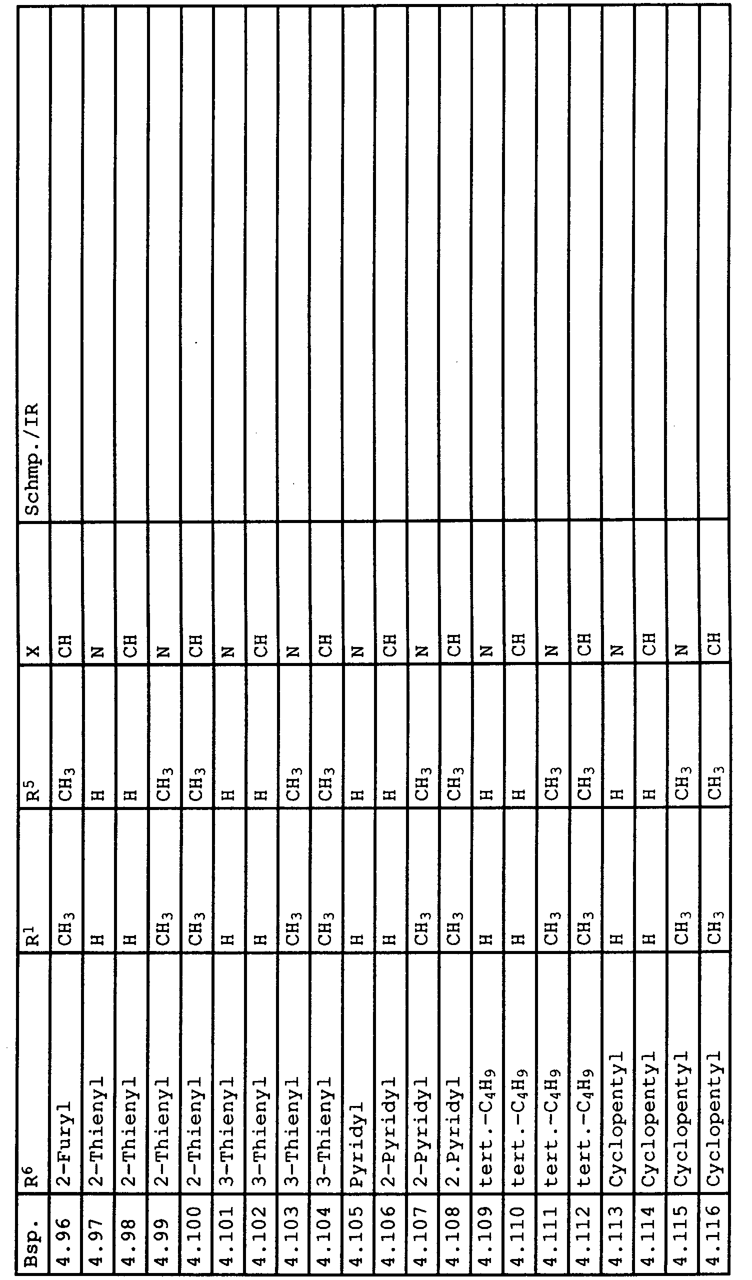

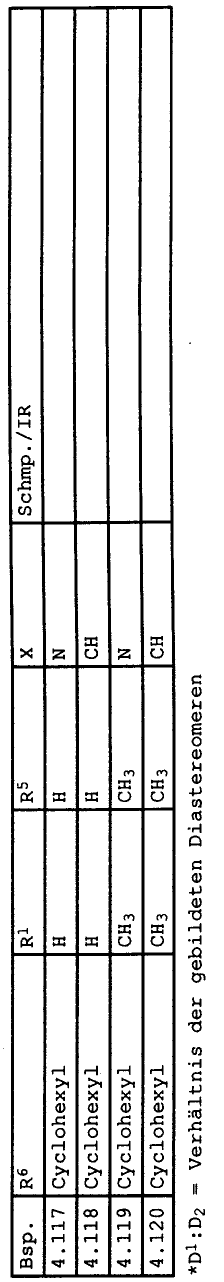

### Vorschrift A2

### 2-(4-Chlorbenzyliden)-3,5,5-trimethyl-cyclohexanon

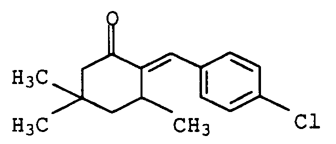

Zu einer Lösung von 763 g (5,45 mol) 3,3,5-Trimethyl-cyclohexanon und 140,5 g (1 mol) 4-Chlorbenzaldehyd werden 10 g Bortrioxyd gegeben und bei 190°C Reaktionstemperatur über 5 Stunden lang Wasser ausgekreist. Bei der anschließenden Destillation des Reaktionsgemisches werden bei 0,2 mbar und 168°c Übergangstemperatur 181 g (69 %) 2-(4-Chlorbenzyliden)-3,5,5-trimethylcyclohexanon erhalten.

### Vorschrift B5

### 3-(4-Chlorbenzyliden)-4,6,6-trimethyl-1-oxa-bicyclo(0,2,5)octan

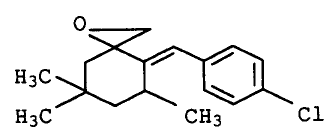

Zu einer Lösung von 40 g (0,15 mol) 2-(4-Chlorbenzyliden)-3,5,5-trimethyl-cyclohexanon in 200 ml Methylenchlorid werden 63 g (0,335 mol) Trimethylsulfoniummethylsulfat und 70 g Natronlauge (konz.) gegeben. Nachdem das Reaktionsgemisch 12 bis 15 Stunden bei Raumtemperatur (20°C) gerührt worden war, werden der Lösung 200 ml Wasser zugesetzt und die organische Phase abgetrennt. Die organische Phase wird zweimal mit Wasser gewaschen, über Natriumsulfat getrocknet und eingeengt.
Ausbeute: 37,4 g (89 %)

### Beispiel 5

### 1-(1,2,4,Triazol-1-yl-methyl)-2-(4-chlorbenzyliden)-3,5,5-trimethyl-cyclohexan∼1∼ol

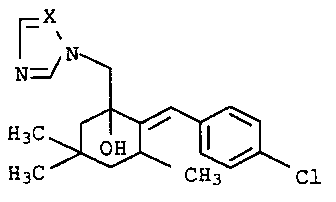

Eine Lösung von 8,8 g (0,128 mol) 1,2,4-Triazol in 100 ml N,N-Dimethylformamid wurde mit 9,8 g Natronlauge (50 gew.-%ig) versetzt und für 30 Minuten auf 50°c erwärmt. Anschließend wurden bei Raumtemperatur (20°c) 27,1 g (0,098 mol) 3∼(4∼Chlorbenzyliden)∼4,6,6∼trimethyl∼1∼oxabicyclo[0,2,5]octan, das in 50 ml N,N∼Dimethylformamid gelöst war, zugetropft und 12 Stunden bei Raumtemperatur gerührt. Anschließend wurden 200 ml Wasser zugegeben und mehrmals mit Methyl-tert.-butylether extrahiert und die organische Phase mit Wasser gewaschen, über Natriumsulfat getrocknet und im Vakuum eingeengt.
Ausbeute: 4,0 g (12 %)
Schmelzpunkt: 88-90°c
Entsprechend Beispiel 5 können die in der Tabelle 5 aufgeführten Verbindungen hergestellt werden.

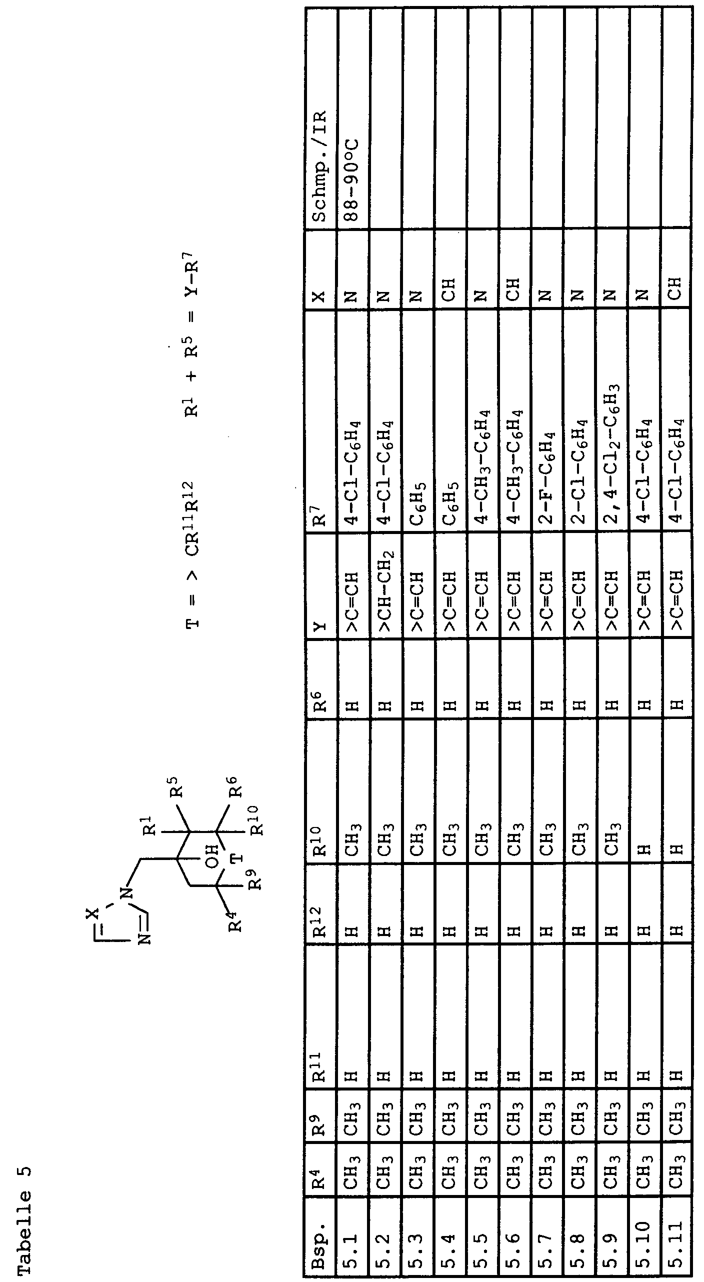

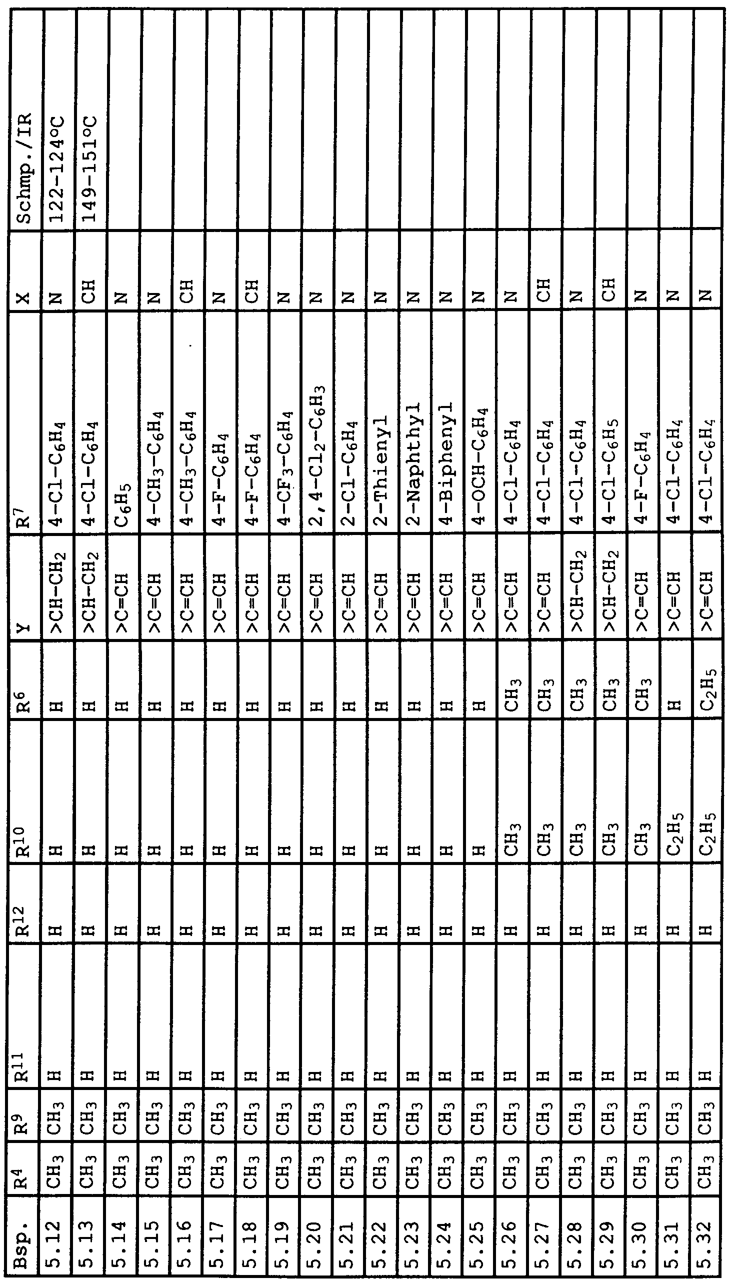

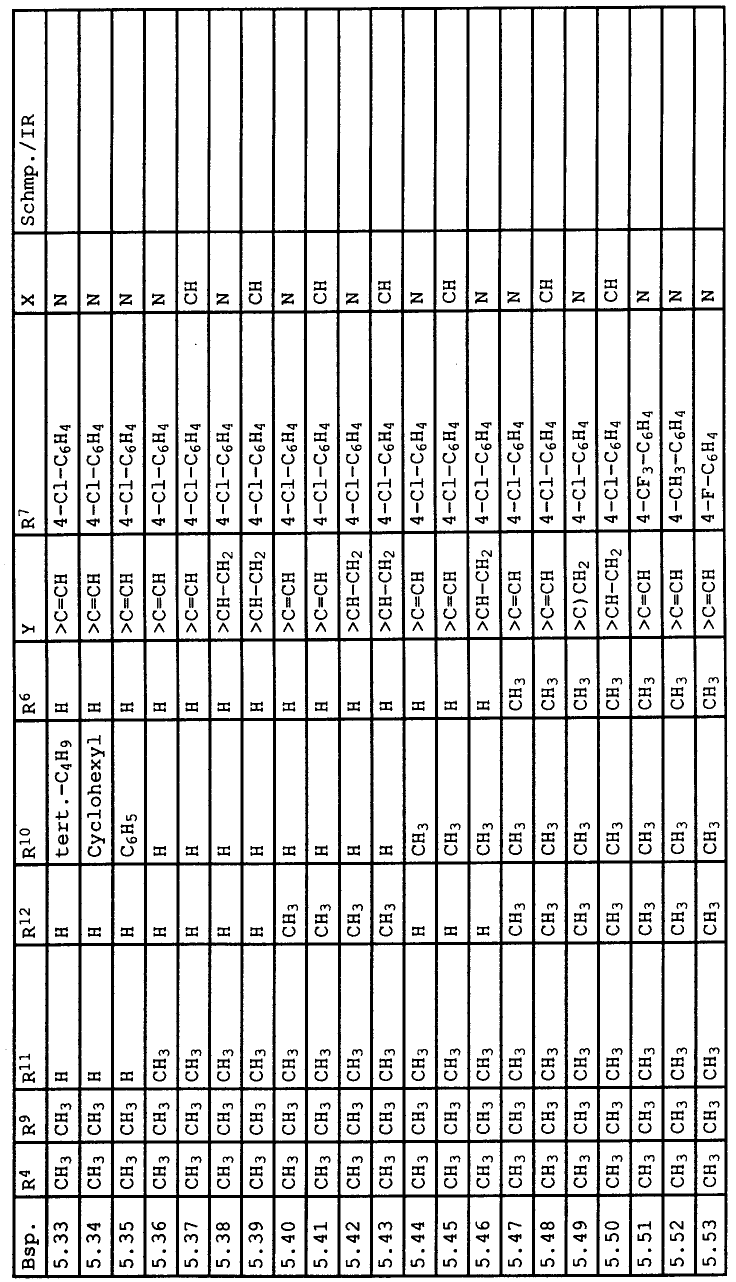

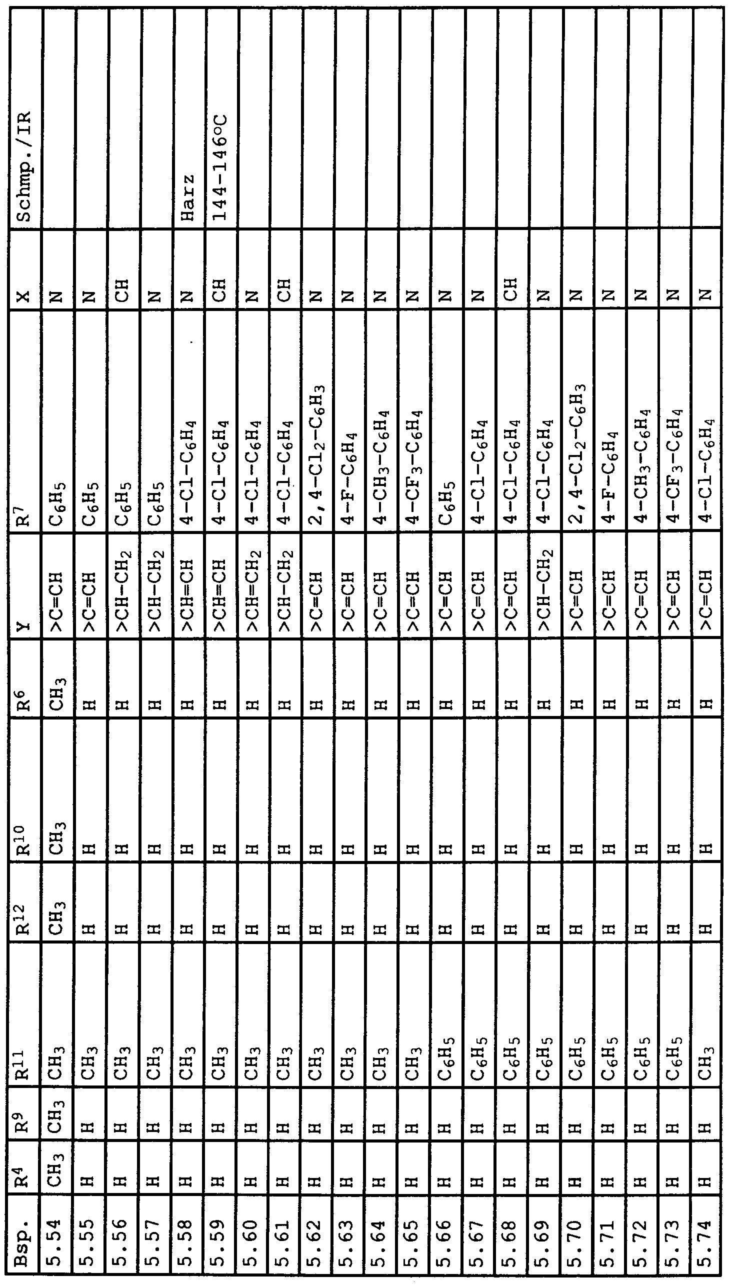

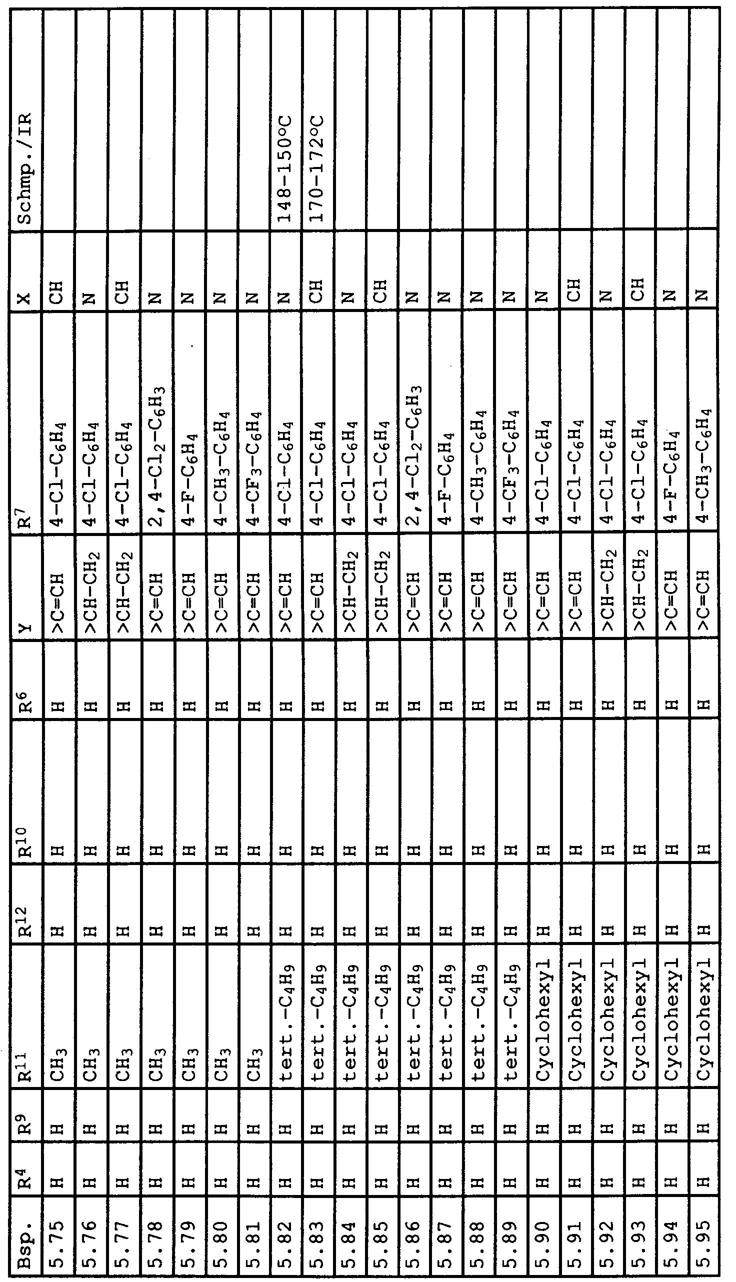

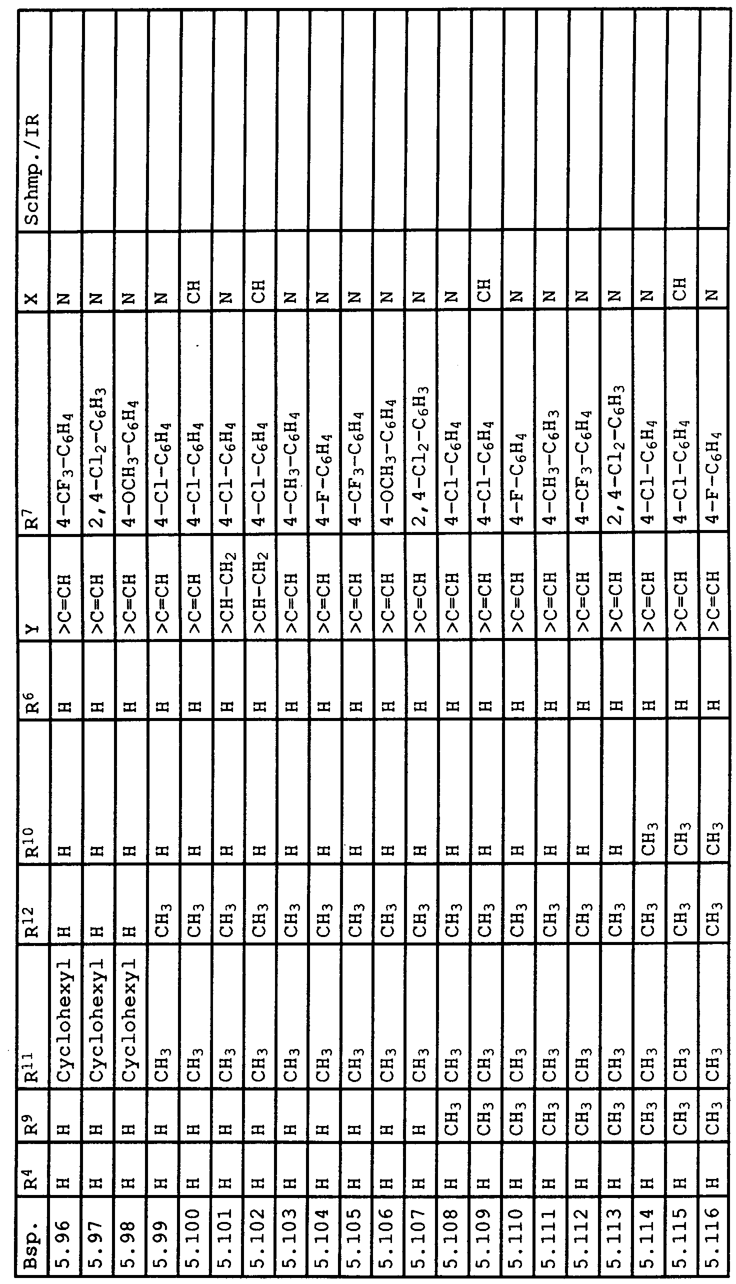

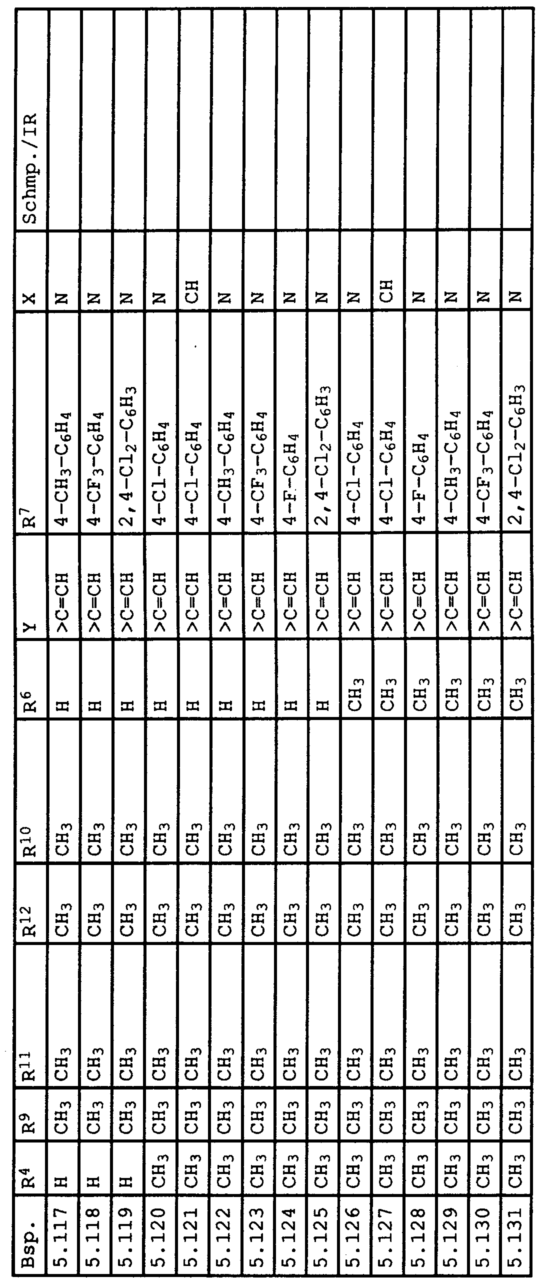

Die Azolylmethylcycloalkanole zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Sie können in die üblichen Formulierungen übergeführt werden, wie Lösungen, Emulsionen, Suspensionen, Stäube, Pulver, Pasten und Granulate. Die Anwendungsformen richten sich nach den Verwendungszwecken; sie sollen in jedem Fall eine feine und gleichmäßige Verteilung des Azolylmethylcycloalkanols gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:
I. eine Lösung aus 90 Gew.-Teilen einer erfindungsgemäßen Verbindung und 10 Gew.-Teilen N-Methyl-α-pyrrolidon, die zur Anwendung in Form kleinster Tropfen geeignet ist;
II. eine Mischung aus 20 Gew.-Teilen einer erfindungsgemäßen Verbindung, 80 Gew.-Teilen Xylol,10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen einer erfindungsgemäßen Verbindung, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen einer erfindungsgemäßen Verbindung, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen einer erfindungsgemäßen Verbindung, 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Die erfindungsgemäßen Mittel können in diesen Anwendungsformen auch zusammen mit anderen Wirkstoffen vorliegen, z.B. mit Herbiziden, Insektiziden, Wachstumsregulatoren, Fungiziden oder auch mit Düngemitteln.

Die Azolylmethylcycloalkanole der Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem
a) von der Pflanzenart und -sorte,
b) von dem Zeitpunkt der Applikation, bezogen auf das Entwicklungsstadium der Pflanze und von der Jahreszeit,
c) von dem Applikationsort und -verfahren (Samenbeize, Bodenbehandlung oder Blattapplikation)
d) von klimatischen Faktoren, z.B. Temperatur, Niederschlagsmenge, außerdem auch Tageslänge und Lichtintensität
e) von der Bodenbeschaffenheit (einschließlich Düngung),
f) von der Formulierung bzw. Anwendungsform des Wirkstoffs und schließlich
g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, werden einige nachstehend erwähnt.
A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.
   Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.
   Von wirtschaftlichem Interesse ist auch die Erhöhung der Standfestigkeit von lageranfälligen Kulturen wie Getreide, Mais, Reis, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.
   Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.
   Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.
   Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.
B. Mit den neuen Mitteln lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.
   Dabei können die Azolylmethylcycloalkanole der Formel I Ertragssteigerungen durch Eingriffe in den pflanzlichen Stoffwechsel bzw. durch Förderung oder Hemmung des vegetativen und/oder des generativen Wachstums verursachen.
C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
   Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wesentlich.
D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.
   - die Öffnungsweite der Stomata reduziert wird
   - eine dickere Epidermis und Cuticula ausgebildet werden
   - die Durchwurzelung des Bodens verbessert wird
   - das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,01 bis 10 kg/ha, bevorzugt 0,05 bis 3 kg/ha, als ausreichend zu betrachten.

Die Formulierungen bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

### Anwendungsbeispiel

Als Vergleichswirkstoff A wurde 1-(1,2,4-Triazol-1-yl-methyl)-2-(4-chlorbenzyl)-cyclohexan-1-ol gewählt, das aus der EP 324 646 bekannt ist.

### Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 bis 22°C und 70 % relativer Luftfeuchtigkeit für weitere 5 Tage kultiviert. Dann wurde das Ausmaß der Symptomentwicklung ermittelt.

| Bonitur: Angabe der befallenen Blattfläche in % | | |
|---|---|---|
| Wirkstoff aus Bsp. | Befall der Blätter nach Applikation von 0,05 %iger wäßriger Wirkstoffaufbereitung | |
| 1.51 | 0 | 0 |
| 2.2 | 1 | 5 |
| 2.3 | 1 | 5 |
| Vergleichswirkstoff A | 3 | 25 |
| Unbehandelt | 4-5 | 65 |

## Patentansprüche

1. Azolylmethylcycloalkanole der allgemeinen Formel I 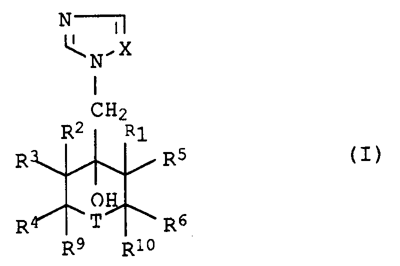 in der
X CH oder N bedeutet;
T (CH₂)ₙ, CR¹¹R¹², O oder S bedeutet,
wobei
n eine ganze Zahl von 1 bis 5 bedeutet; und
R¹¹, R¹² Wasserstoff oder C₁-C₈-Alkyl, Phenyl, Biphenyl, Naphthyl, Heteroaryl, Benzyl, Dioxolanyl oder C₃-C₈-Cycloalkyl bedeutet, wobei jeder dieser Reste ein- bis dreifach durch Halogen, Nitro, Phenoxy, Amino, C₁-C₄- Alkyl, c₁-C₄-Alkoxy oder c₁-c₄-Halogenalkyl substituiert sein kann;
R¹ und R⁵ gleich oder verschieden sind und für Wasserstoff oder C₁-C₄-Alkyl stehen oder
R¹ und R⁵ gemeinsam mit dem C-Atom, dessen Substituenten sie sind, für C=CH-R⁷ oder für CH-Z-R⁷ stehen, wobei
Z CH₂, O, S, SO, SO₂ oder N-R⁸ bedeutet,
R⁷ C₁-C₈-Alkyl, Phenyl, Biphenyl, Naphthyl, Heteroaryl, Benzyl oder C₃-C₈-Cycloalkyl bedeutet, wobei jeder dieser Reste ein- bis dreifach durch Halogen, Nitro, Phenoxy, Amino, C₁ -C₄-Alkyl, C₁-C₄-Alkoxy oder C₁-C₄-Halogenalkyl substituiert sein kann; oder Tetrahydropyranyl bedeutet; wobei
R⁷ für den Fall, daß R¹ und R⁵ gemeinsam mit dem C-Atom, dessen Substituenten sie sind, eine CH-Z-R⁷-Gruppe bedeutet, zusätzlich für Wasserstoff stehen kann; und
R⁸ für Wasserstoff oder C₁-C₄-Alkyl steht;
R² und R³ jeweils für Wasserstoff oder C₁-C₄-Alkyl stehen; oder
R² und R³ gemeinsam mit dem C-Atom, dessen Substituenten sie sind, eine C=CH-R⁷ Gruppe, für den Fall, daß R¹ und R⁵ gemeinsam mit dem C-Atom, dessen Substituenten sie sind, eine C=CH-R⁷ Gruppe bedeuten;
R⁴ Wasserstoff oder C₁-C₄-Alkyl
und
R⁶,R⁹ u.R¹⁰ Wasserstoff oder
C₁-C₈-Alkyl, Phenyl, Biphenyl, Naphthyl, Heteroaryl, Benzyl, Dioxolanyl oder C₃-C₈-Cycloalkyl bedeutet, wobei jeder dieser Reste ein- bis dreifach durch Halogen, Nitro, Phenoxy, Amino, C₁-C₄-Alkyl, C₁-C₄-Alkoxy oder C₁-C₄-Halogenalkyl substituiert sein kann;
ausgenommen Verbindungen,
a) in denen die Reste R¹ bis R¹² gleichzeitig Wasserstoff bedeuten,
b) in denen R¹ und R⁵ gemeinsam mit dem C-Atom, an das sie gebunden sind, für CH-CH₂-(4-Chlorphenyl) oder CH-O-(4-Chlorphenyl) stehen, wenn R² bis R⁴ und R⁶ bis R¹² Wasserstoff bedeuten,
c) in denen R¹ und R⁵ gemeinsam mit dem C-Atom, an das sie gebunden sind, für C=CH-(4-Fluorphenyl) oder für C=CH-(4-Chlorphenyl) stehen, wenn
T CH₂ oder (CH₂)₂ bedeutet,
R² und R³ für Wasserstoff, Cyclohexyl, Phenyl oder C₁- C₄-Alkyl stehen und
R⁴,R⁶,R⁹ und R¹⁰ Wasserstoff bedeuten;
sowie deren pflanzenverträgliche Säureadditionssalze und Metallkomplexe.

2. Azolylmethylcycloalkanole der allgemeinen Formel I gemäß Anspruch 1, in der R⁷ einen ggf. ein- bis dreifach durch Fluor oder Chlor substituierten Phenylrest bedeutet.

3. Verbindung der allgemeinen Formel I gemäß Anspruch 1, in der X für CH steht, R¹ und R⁵ gemeinsam mit dem C-Atom an das sie gebunden sind, für CH-S-(4-Chlorphenyl) stehen, T für CH₂ steht und R² bis R⁴ und R⁶, R⁹ und R¹⁰ Wasserstoff bedeuten.

4. Verbindung der allgemeinen Formel I gemäß Anspruch 1, in der X für CH steht, R¹ und R⁵ sowie R² und R³ jeweils gemeinsam mit dem C-Atom, an das sie gebunden sind, für C=CH-(4-Chlorphenyl) stehen, T für CH₂ steht und R⁴, R⁶, R⁹ und R¹⁰ Wasserstoff bedeuten.

5. Verbindung der allgemeinen Formel I gemäß Anspruch 1, in der X für N steht, R¹ und R⁵ sowie R² und R³ jeweils gemeinsam mit dem C-Atom, an das sie gebunden sind für C=CH-(4-Chlorphenyl) stehen, T für CH₂ steht und R⁴, R⁶, R⁹ und R¹⁰ Wasserstoff bedeuten.

6. Verfahren zur Herstellung von Azolylmethylcycloalkanolen der allgemeinen Formel I gemäß Anspruch 1, dadurch gekennzeichnet, daß man eine Verbindung der allgemeinen Formel II 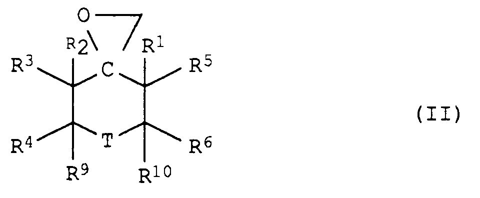 mit einer Verbindung der allgemeinen Formel III 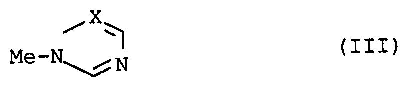 in der Me für ein Wasserstoff- oder ein Metallatom steht, umsetzt und die so erhaltenen Verbindungen ggf. mit pflanzenverträglichen Säuren in ihre Salze oder in ihre Metallkomplexe überführt.

7. Fungizides Mittel enthaltend einen Trägerstoff und eine fungizid wirksame Menge eines Azolylmethylcycloalkanols der allgemeinen Formel I gemäß Anspruch 1.

8. Verfahren zur Bekämpfung von Pilzen, dadurch gekennzeichnet, daß man eine fungizid wirksame Menge eines Azolylmethylcycloalkanols der allgemeinen Formel I gemäß Anspruch 1 auf Pilze oder durch Pilzbefall bedrohte Materialien, Flächen (ausgenommen am tierischen oder menschlichen Körper), Pflanzen oder Saatgüter einwirken läßt.

9. Mittel zur Regulierung des Pflanzenwachstums, enthaltend einen Trägerstoff und eine regulatorisch wirksame Menge eines Azolylmethylcycloalkanols der allgemeinen Formel I gemäß Anspruch 1.

10. Verfahren zur Regulierung des Pflanzenwachstums, dadurch gekennzeichnet, daß man eine regulatorisch wirksame Menge eines Azolylmethylcycloalkanols der allgemeinen Formel I gemäß Anspruch 1 auf Kulturpflanzen oder deren Lebensraum einwirken läßt.

## Claims

1. An azolylmethylcycloalkanol of the general formula I 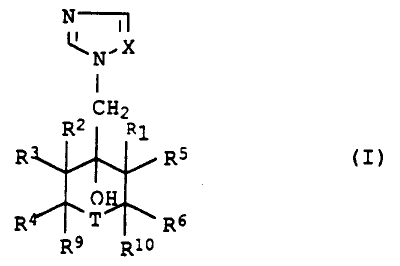 where
X is CH or N;
T is (CH₂)ₙ, CR¹¹R¹², O or S,
where
n is an integer from 1 to 5 and
R¹¹, R¹² are each hydrogen or C₁-C₈-alkyl, phenyl, biphenyl, naphthyl, heteroaryl, benzyl, dioxolanyl or C₃-C₈-cycloalkyl, where each of these radicals may be monosubstituted to trisubstituted by halogen, nitro, phenoxy, amino, C₁-C₄-alkyl, C₁-C₄-alkoxy or C₁-C₄-haloalkyl;
R¹ and R⁵ are identical or different and are each hydrogen or C₁-C₄-alkyl or
R¹ and R⁵ together with the carbon atom of which they are substituents, are C=CH-R⁷ or CH-Z-R⁷,
where
Z is CH₂, O, S, SO, SO₂ or N-R⁸,
R⁷ is C₁-C₈-alkyl, phenyl, biphenyl, naphthyl, heteroaryl, benzyl or C₃-C₈-cycloalkyl, where each of these radicals may be monosubstituted to trisubstituted by halogen, nitro, phenoxy, amino, C₁-C₄-alkyl, C₁-C₄-alkoxy or C₁-C₄-haloalkyl, or is tetrahydropyranyl, and
where R¹ and R⁵ together with the carbon atom of which they are substituents, are CH-Z-R⁷, R⁷ may additionally be hydrogen; and
R⁸ is hydrogen or C₁-C₄-alkyl;
R² and R³ are each hydrogen C₁-C₄-alkyl; or
R² and R³ together with the carbon atom of which they are substituents, are C=CH-R⁷ where R¹ and R⁵ together with the carbon atom of which they are substituents, are C=CH-R⁷;
R⁴ is hydrogen or C₁-C₄-alkyl
and
R⁶, R⁹, and R¹⁰ are each hydrogen or
C₁-C₈-alkyl, phenyl, biphenyl, naphthyl, heteroaryl, benzyl, dioxolanyl or C₃-C₈-cycloalkyl, where each of these radicals may be monosubstituted to trisubstituted by halogen, nitro, phenoxy, amino, C₁-C₄-alkyl, C₁-C₄-alkoxy or C₁-C₄-haloalkyl;
with the exception of compounds
a) in which R¹ to R¹² are simultaneously hydrogen,
b) in which R¹ and R⁵ together with the carbon atom to which they are bonded, are CH-CH₂-(4-chlorophenyl) or CH-O-(4-chlorophenyl) where R² to R⁴ and R⁶ to R¹² are hydrogen,
c) in which R¹ and R⁵, together with carbon atoms to which they are bonded, are C=CH-(4-fluorophenyl) or C=CH-(4-chlorophenyl) when
T is CH₂ or (CH₂)₂,
R² and R³ are each hydrogen, cyclohexyl, phenyl or C₁-C₄-alkyl and
R⁴, R⁶, R⁹ and R¹⁰ are each hydrogen;
and the plant-tolerated acid addition salts and metal complexes thereof.

2. An azolylmethylcycloalkanol of the general formula I as claimed in claim 1, where R⁷ is phenyl which is unsubstituted or monosubstituted to trisubstituted by fluorine or chlorine.

3. A compound of the general formula I as claimed in claim 1, in which X is CH, R¹ and R⁵ together with the carbon atom to which they are bonded, are CH-S-(4-chlorophenyl), T is CH₂ and R² to R⁴ and R⁶, R⁹ and R¹⁰ are each hydrogen.

4. A compound of the general formula I as claimed in claim 1, in which X is CH, R¹ and R⁵ on the one hand and R² and R³ on the other hand together with the carbon atom to which they are bonded, are in each case C=CH-(4-chlorophenyl), T is CH₂ and R⁴, R⁶, R⁹ and R¹⁰ are each hydrogen.

5. A compound of the general formula I as claimed in claim 1, in which X is N, R¹ and R⁵ on the one hand and R² and R³ on the other hand together with the carbon atom to which they are bonded, are in each case C=CH-(4-chlorophenyl), T is CH₂ and R⁴, R⁶, R⁹ and R¹⁰ are each hydrogen.

6. Process for the preparation of an azolylmethylcycloalkanol of the general formula I as claimed in claim 1, wherein a compound of the general formula II 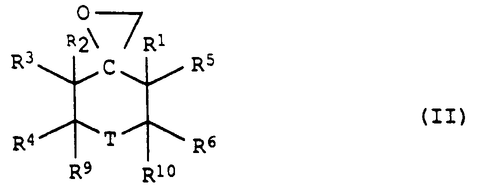 is reacted with a compound of the general formula III 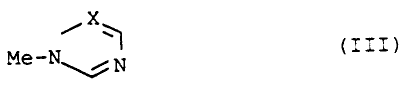 where Me is hydrogen or a metal atom, and the compound thus obtained is converted, if desired, into its salts or into its metal complexes with plant-tolerated acids.

7. A fungicide containing a carrier and fungicidal amount of an azolylmethylcycloalkanol of the general formula I as claimed in claim 1.

8. A method for controlling fungi, wherein a fungicidal amount of an azolylmethylcycloalkanol of the general formula I as claimed in claim 1 is allowed to act on fungi or on materials, surfaces (with the exception of animal or human bodies), crop plants or seeds threatened by fungal attack.

9. A plant growth regulator containing a carrier and a regulatory amount of an azolylmethylcycloalkanol of the general formula I as claimed in claim 1.

10. A method for regulating plant growth, wherein a regulatory amount of an azolylmethylcycloalkanol of the general formula I as claimed in claim 1 is allowed to act on crops or their habitats.

## Revendications

1. Azolylméthylcycloalkanoles de formule générale I 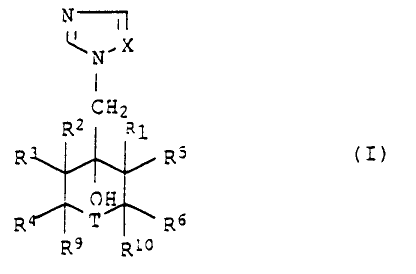 dans laquelle
X représente CH ou N
T (CH₂)ₙ, CR¹¹R¹², O ou S
n représente un nombre entier de 1 à 5 et
R¹¹, R¹² représentant hydrogène ou alkyle en C1-C8, phényle, biphényle, napthyle, hétéroaryle, benzyle, dioxolanyle ou cycloalkyle en C3-C8, chacun de ces restes pouvant être substitué une à trois fois par halogène, nitro, phénoxy, amino, alkyle en C1-C4, alcoxy en C1-C4 ou halogénalkyle en C1-C4 ;
R¹ et R⁵ étant identiques ou différents et mis pour hydrogène ou alkyle en C1-C4 ou
R¹ et R⁵ ensemble avec l'atome C dont ils sont des substituants étant mis pour C=CH-R⁷ ou par CH-Z-R⁷,
Z représente CH₂, O, S, SO, SO₂ ou N-R⁸
R⁷ alkyle en C1-C8, phényle, biphényle, naphtyle, hétéroaryle, benzyle ou cycloalkyle en C3-C8, chacun de ces restes pouvant être substitué une à trois fois par halogène, nitro, phénoxy, amino, alkyle en C1-C4, alcoxy en C1-C4 ou halogènalkyle en C1-C4 ou représentant tétrahydropyranyle ;
R⁷ pouvant être mis en outre pour hydrogène dans le cas où R¹ et R⁵ ensemble avec l'atome C dont ils sont des substituants représente un groupe CH-Z-R⁷, et
R⁸ étant mis pour hydrogène ou alkyle en C1-C4 ;
R² et R³ étant mis chacun pour hydrogène ou alkyle en C1-C4 ; ou
R² et R³ représentant ensemble avec l'atome C dont ils sont des substituants un groupe C=CH-R⁷ dans le cas où R¹ et R⁵ ensemble avec l'atome C dont ils sont substituants représentent un groupe C=CH-R⁷ ;
R⁴ représentant hydrogène ou alkyle en C1-C4
et
R⁶, R⁹ et R¹⁰ représentent hydrogène ou
alkyle en C1-C8, phényle, biphényle, naphtyle, hétéroaryle, benzyle, dioxolanyle ou cycloalkyle en C3-C8, chacun de ces restes pouvant être substitué une à trois fois par halogène, nitro, phénoxy, amino, alkyle en C1-C4, alcoxy en C1-C4 ou halogénoalkyle en C1-C4 ;
à l'exclusion des composés
a) dans lesquels les restes R¹ à R¹² représentent simultanément l'hydrogène
b) dans lesquels R¹ et R⁵, ensemble avec l'atome C auquel ils sont liés, sont mis pour CH-CH₂-(4-chlorophényle) ou CH-O-(4-chlorophényle) quand R² à R⁴ et R⁶ à R¹² représentent hydrogène
c) dans lesquels R¹ et R⁵, ensemble avec l'atome C auquel ils sont liés, sont mis pour C=CH-(4-fluorophényle) ou pour C=CH-(4-chlorophényle), quand
T représente CH₂ ou (CH₂)₂,
R² et R³ représentent hydrogène, cyclohexyle, phényle ou alkyle en C1-C4 et
R⁴, R⁶, R⁹ et R¹⁰ représentent hydrogène
ainsi que leurs sels d'addition d'acide et complexes métalliques acceptables pour les plantes.

2. Azolylméthylcycloalkanole de formule générale I selon la revendication 1, dans laquelle R⁷ représente un reste phényle éventuellement substitué une à trois fois par fluor ou chlore.

3. Composé de formule générale I selon la revendication 1, dans laquelle R¹ et R⁵, ensemble avec l'atome C auquel ils sont liés, sont mis pour CH-S-(4-chlorophényle), X est mis pour CH, T est mis pour CH₂ et R² à R⁴ et R⁶, R⁹ et R¹⁰ représentent hydrogène.

4. Composé de formule générale I selon la revendication 1, dans laquelle X est mis CH, R¹ et R⁵ ainsi que R² et R³, chacun ensemble avec l'atome C auquel ils sont liés, sont mis pour C=CH-(4-chlorophényle), T est mis pour CH₂ et R⁴, R⁶, R⁹ et R¹⁰ représentent hydrogène.

5. Composé de formule générale I selon la revendication 1, dans laquelle X est mis pour N, R¹ et R⁵ ainsi que R² et R³, chacun ensemble avec l'atome C auquel ils sont liés, sont mis pour C=CH-(4-chlorophényle), T est mis pour CH₂ et R⁴, R⁶, R⁹ et R¹⁰ représentent hydrogène.

6. Procédé de préparation d'azolylméthylcycloalkanole de formule générale I selon la revendication 1, caractérisé par le fait que l'on fait réagir un composé de formule générale II 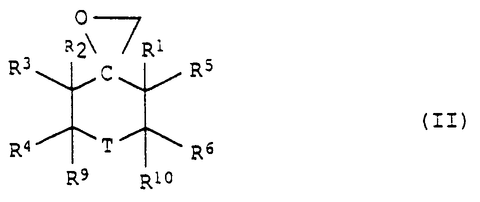 avec un composé de formule générale III 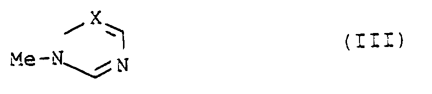 dans laquelle Me est mis pour un atome d'hydrogène ou un atome métallique, et on transforme le composé ainsi obtenu, éventuellement avec des acides acceptables par les plantes en leurs sels ou en leurs complexes métalliques.

7. Fongicide cOntenant un support et une quantité efficace comme fongicide d'un azolylméthylcycloalkanole de formule générale I selon la revendication 1.

8. Procédé pour lutter contre les champignons, caractérisé par le fait que l'on fait réagir une quantité efficace comme fongicide d'un azolylméthylcycloalkanole de formule générale I selon la revendication 1 sur les champignons ou sur les matières, surfaces (sauf sur les corps animaux ou humains), plantes ou semences menacés par des champignons.

9. Agent de régularisation de la croissance des plantes, contenant un support et une quantité à effet régulatrice d'un azolylméthylcycloalkanole de formule générale I selon la revendication 1.

10. Procédé de régularisation de la croissance des plantes, caractérisé par le fait que l'on fait agir sur les plantes de culture ou leur biotope une quantité à effet régulateur d'un azolylméthylcycloalkanole de formule générale I selon la revendication 1.
